(19) Europäisches Patentamt / European Patent Office / Office européen des brevets

(11) **EP 4 492 051 A1**

(12) **EUROPÄISCHE PATENTANMELDUNG**


(43) Veröffentlichungstag:
**15.01.2025 Patentblatt 2025/03**

(21) Anmeldenummer: **23185000.9**

(22) Anmeldetag: **12.07.2023**

(51) Internationale Patentklassifikation (IPC):
*G01N 29/04* (2006.01) *G01N 29/14* (2006.01)
*G01N 29/22* (2006.01) *G01N 29/44* (2006.01)
*G01N 29/46* (2006.01) *G01N 33/38* (2006.01)

(52) Gemeinsame Patentklassifikation (CPC):
**G01N 29/046; G01N 29/14; G01N 29/228; G01N 29/4436; G01N 29/4481; G01N 29/46; G01N 33/383;** G01N 2291/0232; G01N 2291/0251; G01N 2291/02827

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**



(71) Anmelder: **KIMA Process Control GmbH**
**52428 Jülich (DE)**

(72) Erfinder:
- **Kalkert, Peter**
  **52428 Jülich (DE)**
- **Kalkert, Matthias**
  **52428 Jülich (DE)**

(74) Vertreter: **Heuking Kühn Lüer Wojtek PartGmbB**
**Georg-Glock-Straße 4**
**40474 Düsseldorf (DE)**


(54) **KLINKERHÄRTEERKENNUNG MITTELS AUSWERTUNG EINES AKUSTISCHEN SIGNALS**


(57) Die Erfindung betrifft eine Vorrichtung (50) zur Bestimmung einer Härte (H) von Zementklinker (ZK) in einem Zementwerk (20) durch Auswertung eines akustischen Akustisches Signals (AS) beim Aufprall des Zementklinkers auf einer Unterlagen (12) nach dem Verlassen eines Drehrohrofens (9), einem Zementwerk mit einer solchen Vorrichtung, ein entsprechendes Verfahren (200) unter Verwendung dieser Vorrichtung sowie einen Datenträger (300) mit einem darauf gespeicherten Softwareprogramm (301) zur Installation in einer Auswerteeinheit (70) einer solchen Vorrichtung zur zumindest teilweise Ausführung eines solchen Verfahrens. Im Zementwerk fällt der Zementklinker aus dem Drehrohrofen auf eine Unterlage und versetzt diese durch den Aufschlag in Schwingungen, was in einem für die jeweilige Härte charakteristischen akustischen Signal (AS) umfassend mindestens eine für die Härte charakteristische Frequenzverteilung (FV) oder Frequenz-Zeit-Verteilung (FZV) oder Zeitsignal (ZV) resultiert, wobei die Vorrichtung (50) einen oder mehrere Sensoren (60) und eine Auswerteeinheit (70) umfasst, wobei die Sensoren (60) dazu vorgesehen sind, das jeweilige akustische Signal (AS) aufzunehmen und die Auswerteeinheit (70) dazu vorgesehen ist, aus der aufgenommenen Frequenzverteilung (FV) oder der aufgenommenen Frequenz-Zeit-Verteilung (FZV) oder dem aufgenommenen Zeitsignal (ZV) zumindest die Härte (H) des Zementklinkers (ZK) zu bestimmen.


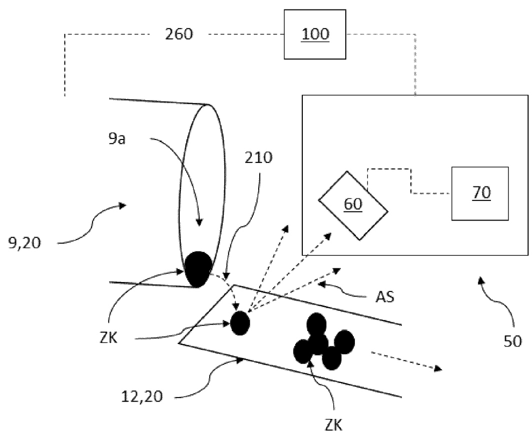


Fig.2

EP 4 492 051 A1

## Beschreibung

### Gebiet der Erfindung

Die Erfindung betrifft eine Vorrichtung zur Bestimmung einer Härte von Zementklinker in einem Zementwerk durch Auswertung eines akustischen Signals beim Aufprall des Zementklinkers auf einer Unterlage nach dem Verlassen des Drehofens, ein Zementwerk mit einer solchen Vorrichtung und ein entsprechendes Verfahren unter Verwendung dieser Vorrichtung.

### Hintergrund der Erfindung

Beim Zementherstellungsprozess wird Kalkgestein (CaCO3) aus dem Steinbruch mittels eines Brechers zerkleinert. Dem zerkleinerten Kalkstein werden in ein Mischbettsilo für den späteren Sinterprozess nötige Stoffe hinzuzufügt und das Material homogenisiert. Anschließend wird das homogenisierte Material in einer Rohmühle gemahlen und in Silos erneut homogenisiert. Von dort aus gelangt das nun Rohmehl genannte Material in den sogenannten Zyklonvorwärmer, der das Rohmehl mit Drehrohrofenabgasen und dem Calcinator auf eine Temperatur von ca. 800 °C erwärmt, um es zu entsäuern. Nach der Entsäuerung durchläuft das Rohmehl einen um die Längsachse rotierenden Drehrohrofen. Der Ofen besitzt eine leichte Neigung von ca. 3° bis 4°. Betrieben wird der Ofen mit einer ca. 2000 °C heißen Flamme, die das Rohmehl auf ca. 1400 °C bis 1450 °C erhitzt. Bei diesen Temperaturen sintert das Rohmehl und die nachfolgende chemische Reaktion läuft ab:

$$\mathrm{CaCO3} \xrightarrow{+\,\text{Wärme}} \mathrm{CaO + CO2}.$$

Bei diesem Vorgang entsteht der Zementklinker. Der frisch gebrannte Zementklinker verlässt den Drehrohrofen bei Temperaturen von 1.200 °C bis 1.250 °C mit einer von den Prozessen im Drehrohrofen abhängigen Härte (Klinkerhärte) und wird auf unter 200 °C abgekühlt. Die Abkühlung des Zementklinkers wird über einem Rostkühler relativ schnell durchgeführt, um chemische Umwandlungsprozesse bestimmter Klinkerphasen zu vermeiden. Eine wesentliche Aufgabe des Rostkühlers ist die Wärmerückgewinnung, bei der die Kühlluft die Wärme des heißen Zementklinkers aufnimmt, die dann im Ofen als vorgewärmte Verbrennungsluft, die sogenannte Sekundärluft, oder in der Mühle als Trockenluft eingesetzt wird. Bei dem Rostkühler wird der Zementklinker auf einer als Rost ausgeführten Unterlage, die aus luftdurchlässigen Platten besteht, im Kreuzstrom zur Kühlluft transportiert und zu einer Zementmühle weitertransportiert, in der der Zementklinker unter Zugabe weiterer Materialien, die den Zementtyp bestimmen, zu Zement gemahlen wird. Bei diesen Zuschlagsmaterialien handelt es sich zum Beispiel um Hüttensand. Der gemahlene Zementklinker läuft vor der Lagerung noch über den sogenannten Sichter. Dessen Aufgabe ist es, zu große Partikel aus dem Mahlvorgang an den Anfang des Mahlvorgangs zu schicken. Die Partikel verbleiben im Mahlkreislauf, bis die maximale Korngröße unterschritten wird.

Die Klinkerhärte ist von vielen Faktoren, aber maßgeblich von der Temperatur im Drehrohrofen abhängig. Der Kalkstein und die Flamme beeinflussen die Regelparameter. Bedingt durch ihre Zusammensetzung aus verschiedenen Materialien schwankt die Temperatur der Flamme. In der Regel wird die Flamme mit Kohle, Hausmüll als Sekundärbrennstoff und Sekundärluft betrieben. Allerdings ist die Temperatur der Sekundärluft nicht konstant und auch die Effizienz des Sekundärbrennstoffs schwankt, da sie u.a. seiner Feuchtigkeit anhängig ist. Die Qualität und Nutzbarkeit des Zements hängt von der Klinkerhärte ab. Beispielsweise kann zu weicher Zementklinker in der Produktion nicht verwendet werden. Der daraus entstehende Zement wäre zu reaktiv und löst sich mit der Zeit auf. Zu harter Zementklinker hingegen ist schwerer zu mahlen, was den Mahlvorgang in die Länge zieht. Außerdem ist diese Art Zementklinker für eine qualitativ gute Verarbeitung zu Beton nicht reaktiv genug.

Im Stand der Technik wird die Klinkerhärte durch das sogenannte Auslitern (Litergewicht) bestimmt. Hierbei wird ein Gefäß bekannten Volumens mit Zementklinker gefüllt und gewogen. Im Anschluss wird das mit Zementklinker gefüllte Gefäß mit Wasser aufgefüllt und erneut gewogen. Damit lässt sich die Klinkerhärte als mittlere Dichte der Zementklinker aus der Testmenge bestimmen. Dieser Vorgang zur Ermittlung der Klinkerhärte dauert länger als der Mahlvorgang. Darüber hinaus führen besonders dichte oder poröse Zementklinker zu Messungenauigkeiten, da Gaseinschlüsse nicht berücksichtigt werden. Dieser Bestimmungsprozess ist somit ein zeitlich nachgeschalteter Prozess, der zudem zeitlich aufwendig ist. Bis zum Erhalt des Ergebnisses der Klinkerhärte wird im Drehrohrofen weiter kontinuierlich Zementklinker erzeugt, der möglicherweise ebenfalls aufgrund der falschen Klinkerhärte verloren ist. Somit kann gegebenenfalls eine große nicht nutzbaren Menge an Zementklinker bei dieser Art der Klinkerhärtenmessung entstehen. Der Zement wird in Abhängigkeit seiner Härte in unterschiedliche Silos einsortiert. Zu harter oder zu weicher Zementklinker ist Ausschuss und kann nicht verwendet werden. Eine schnellere Bestimmung würde an dieser Tatsache zwar nichts ändern, jedoch würde die produzierte Menge des nicht nutzbaren Zements auf ein Minimum reduziert werden, da der Prozess zeitnah

nachjustiert werden könnte.

Daher ist es überaus wünschenswert, die Klinkerhärte möglichst unmittelbar nach der Herstellung im laufenden Betrieb zu detektieren, um im Herstellprozess sofort auf Härteschwankungen reagieren zu können und die Steuerparameter des Drehrohrofenprozesses unmittelbar anpassen zu können.

## Zusammenfassung der Erfindung

Aufgabe der Erfindung ist es, eine Vorrichtung bzw. ein Verfahren bereitzustellen, mit dem es möglich ist, die Härte des Zementklinkers unmittelbar nach dessen Herstellung im laufenden Betrieb zu detektieren, um damit nachfolgend im Herstellprozess sofort auf Schwankungen oder ungünstige Härten des Zementklinker reagieren zu können und ggf. die Steuerparameter des Drehrohrofenprozesses unmittelbar anpassen zu können.

Diese Aufgabe wird gelöst durch eine Vorrichtung zur Bestimmung einer Härte von Zementklinker in einem Zementwerk, wobei der Zementklinker, der einen Drehrohrofen des Zementwerks verlässt, auf eine Unterlage fällt und durch einen Aufschlag auf die Unterlage diese in Schwingungen versetzt, was in einem für die jeweilige Härte charakteristischen akustischen Signal umfassend mindestens eine für die Härte charakteristische Frequenzverteilung oder Frequenz-Zeit-Verteilung oder Zeitsignal resultiert, wobei die Vorrichtung einen oder mehrere Sensoren und eine Auswerteeinheit umfasst, wobei die Sensoren dazu vorgesehen sind, das jeweilige akustische Signal aufzunehmen und die Auswerteeinheit dazu vorgesehen ist, aus der aufgenommenen Frequenzverteilung oder der aufgenommenen Frequenz-Zeit-Verteilung oder dem aufgenommenen Zeitsignal zumindest die Härte des Zementklinkers zu bestimmen.

Der Begriff "Unterlage" bezeichnet dabei Unterlagen, die dazu dienen, dass sich der über 1000°C heiße Zementklinker auf der Unterlage zur weiteren Verarbeitung auf wenige 100°C abkühlt. Diese Unterlagen können dabei als Rost ausgeführt sein, damit Kühlluft den Zementklinker durchströmen kann. Diese Unterlagen tragen eine große Masse an Zementklinker und daher ein hohes Gewicht. Sie sind daher auf einem Untergrund fest montiert und geben beim Aufprall des Zementklinkers auf die Unterlage nicht nach oder federn den Aufprall zumindest nicht merklich ab. Dadurch gerät die Unterlage durch den Aufprall des aus dem Drehofen herabfallenden Zementklinkers in Schwingungen, wodurch das zu detektierende akustische Signal erzeugt wird. Eine nachgebende oder abfedernde Unterlage würde kein oder ein zu geringes akustisches Signal erzeugen, das zudem durch die Abfederung oder Nachgeben der Unterlage nicht reproduzierbar für Zementklinker mit derselben Härte wäre, sodass das voranstehende Messprinzip keine reproduzierbaren Ergebnisse liefern würde. Das akustische Signal kann sich über die Luft als Luftschallsignal und/oder über die Unterlage als Körperschallsignal ausbreiten und den oder die Sensoren erreichen, die entweder in einer Entfernung zur Unterlage oder in mechanischem Kontakt mit der Unterlage angeordnet sind. Der oder die Sensoren können beispielsweise ein oder mehrere Elemente aus der Gruppe Klopfsensor oder Luftschallmikrofon sein. Bei einem Luftschallmikrofon ist das akustische Signal als Luftschallsignal unabhängig von der Beschaffenheit und Aufhängung der Unterlage. Bei einem Klopfsensor misst dieser das akustische Signal als Körperschallsignal, das sich durch den Aufprall des Zementklinkers auf die Unterlage in der Unterlage ausbreitet. Dieses Signal erleidet weniger Störungen gegenüber dem Luftschallsignal. Da ein Luftschallmikrofon neben dem akustischen Signal alle weiteren Schallsignale der Umgebung mit aufnimmt, muss das eigentliche akustische Signal von den Störsignalen getrennt werden. Das Körperschallsignal ist dagegen weniger von der Umgebung gestört. Daher werden Klopfsensoren als Sensoren zur Aufnahme des akustischen Signals als Körperschallsignal bevorzugt.

Die Erfindung zur Bestimmung der Härte von Zementklinker setzt voraus, dass der Zementklinker in einem Drehrohrofen gebrannt wird und anschließend aus dem Drehrohrofen auf eine Unterlage fällt. Die Härte des Zementklinkers entscheidet, ob der Zementklinker gemäß Fachjargon unter Zementbrennern ein "guter oder schlechter Brand " ist. Ein guter oder schlechter Brand beurteilt sich nach den späteren Eigenschaften des nach dem Mahlen entstehenden Zementes. Die Erstarrung, Reaktionsgeschwindigkeit und das entstehende Gefüge sind vor allem durch die Zusatzstoffe des Zementes festgelegt. Die eigentliche Qualität des Zementklinkers dagegen geht einher mit der Eigenschaft der Raumbeständigkeit des Zementklinkers. Die Raumbeständigkeit des Zements beschreibt dessen Ausdehnung während dem Aushärten, hierbei sind vor allem der so genannte Frei-Kalk-Gehalt sowie der Periklas-Gehalt entscheidend. Da die Erstarrung vor allem durch die Zusatzstoffe, in Bezug auf die Reaktionsgeschwindigkeit und das Gefüge, eingestellt werden kann, wird die Qualitätsprüfung des Zementklinkers durch seine Eigenschaft der Raumbeständigkeit bestimmt. Die Raumbeständigkeit des Zements beschreibt dessen Ausdehnung während dem Aushärten. Daher sollte der Anteil an freiem Kalk ($CaO$) und an Perikal ($MgO$) kleiner 2 Gew.% bzw. kleiner 5 Gew.% sein. Freier Kalk ist der im Drehrohrofen nicht in die feste Lösung übergehende Bestandteil an $CaO$, welcher dann zusätzlich zu den anderen Klinkerphasen im gebrannten Material vorliegt. Das $MgO$, welches als eigenständige Phase vorliegt, sowie der Freie Kalk, bestimmen den Einfluss der Volumenzunahme in der späteren Verarbeitung des Zementes mit Wasser. Der Frei-Kalk-Gehalt, sowie der Gehalt an $MgO$, ist daher ein wesentliches Qualitätsmerkmal des Zementklinkers. Das akustische Signal hängt wiederum von der inneren Zusammensetzung des Zementklinkers und somit von seinen dadurch bedingten mechanischen Eigenschaften ab und liefert daher auswertbare Daten zur inneren Zusammensetzung.

Aus der Fallstrecke erhält der Zementklinker einen Impuls. Eine Impulsänderung bezüglich der Fallrichtung stellt

sich dann in Folge des mechanischen Kontakts mit der Unterlage ein. Die zeitliche Differentiation dieses Impulses entspricht dann einer Kraft, die auf die Unterlage wirkt. Mit Annahme der Impulserhaltung muss damit die Kraft/Zeit-Kurve beim Aufprall einer Funktion entsprechen, für die das Integral die erhaltende Größe, also in diesem Falle dem Impuls, entspricht. Stellt man die Kraft/Zeit-Funktion als Gaußkurve mit einer maximalen Kraft Fmax und mit einer Breite $\sigma$ dar, so kann physikalisch die Breite der Funktion mit der Härte des Zementklinkers korreliert werden. Ein weiches Material der gleichen Masse würde eine flacherer Gauß-Kurve bedeuten, entsprechend einer größeren Breite $\sigma$. Die Schwingungsanregung der Unterlage kann verstanden werden über die Faltung eines Eingangsimpulses mit einer entsprechenden Übertragungsfunktion der Unterlage. Der Eingang des Systems ist somit eine Kraft, der Ausgang eine Verschiebung x eines Punktes der Unterlage. Die Verschiebung x am örtlich fixierten Punkt der Unterlage drückt sich in einer entsprechenden Schwingung aus, welche aus den sich ausbildenden Wellen auf der Unterlage resultiert. Diese Wellen entsprechen dem akustischen Signal, welches über die Sensoren entsprechend detektiert wird. Da Wellen Gebilde in Ort und Zeit sind, verändert sich die Amplitude also auch bei einem Wechsel des Betrachtungspunktes. Die Betrachtung der Schwingung kann an einem örtlich fixierten Punkt auf einer schwingenden Unterlage stattfinden. Im fixierten Punkt können nun mehrere harmonische Schwingungen vorliegen. Unterschiedlich "harte" Zementklinker-Materialien verändern die Frequenzverteilung bzw. die Frequenz-Zeit-Verteilung bzw. das Zeitsignal. Ein zeitlicher schmaler Kraft-Impuls sorgt für eine breite Anregung der Schwingungsmoden der Unterlage, auch der der höheren Schwingungsmoden. Ein zeitlich breiter Kraft-Impuls entspricht einem im Frequenzraum schmalen Puls und damit einer schwachen Anregung höherer Schwingungsmoden. Ein harter Zementklinker resultiert in einem ins Verhältnis zu einem weichen Zementklinker gesetzten, schmaleren Impuls im Zeitbereich. Ein schmaler mechanischer Impuls im Zeitbereich regt eine breitere Frequenzverteilung an als ein zeitlich breiterer mechanischer Impuls. Die Frequenz-Zeit-Verteilung weist dagegen eine für die Härte charakteristische Abfolge von Frequenzen über die Zeit auf. Das Zeitsignal weist einen für die Härte charakteristischen Anstieg auf, der umso steiler ausfällt, je härter der Zementklinker ist.

Damit ist es möglich, über Aufnahme der akustischen Signale und deren nachfolgende Auswertung die Härte des jeweiligen, gerade auf die Unterlage gefallenen Zementklinkers zu bestimmen. Da die Einflussgrößen zur Variation der Härte des Zementklinkers grundsätzlich bekannt sind, ermöglichen diese eine praktisch in Echtzeit stattfindende Bestimmung der Härte des Zementklinkers, um sofort auf ungünstige Klinkerhärten mittels Anpassung der Produktionsparameter reagieren zu können. Beim Stand der Technik werden Proben des Zementklinkers aus dem laufenden Betrieb entnommen und erst zeitaufwendig im Labor vermessen, was einen beträchtlichen Zeitversatz zwischen dem Herstellungszeitpunkt des untersuchten Zementklinkers und dem Zeitpunkt des Messergebnisses für diesen Zementklinkers bedeutet. Gegebenenfalls ist die gesamte in diesem Zeitintervall hergestellte Menge an Zementklinker Ausschuss durch zu weichen oder zu harten Zementklinker. Die erfindungsgemäße Methode und die entsprechend betriebene Vorrichtung können den Ausschuss stark verringern oder gar vermeiden, indem die Prozessparameter in Echtzeit und damit rechtzeitig angepasst werden, um erst gar keinen Ausschuss entstehen zu lassen. Die Auswerteeinheit umfasst dazu ein oder mehrere Prozessoren, die die für die Auswertung der akustischen Signale benötigten Algorithmen ausführen können sowie einen geeigneten Datenspeicher, um Datensätze und Ergebnisse zu speichern. Die Auswerteeinheit kann dabei mittels Datenverbindungen mit den Komponenten der Vorrichtung und gegebenenfalls auch der steuernden Komponenten des Zementwerks verbunden sein.

Zusätzlich vermeidet die vorliegende Erfindung die aufwendige Bestimmung des Litergewichts separat von der laufenden Produktion des Zementklinkers mittels Laboranalyse von entnommenen Zementklinkerproben. Somit kann mit der vorliegenden Erfindung die Menge an zu untersuchenden Proben an Zementklinker aus der laufenden Produktion stark verringert werden und damit auch der separate Messaufwand parallel zur Echtzeitmessung gesenkt werden.

In einer Ausführungsform ist die Auswerteeinheit dazu vorgesehen, zum Beispiel mittels einer Fouriertransformation oder einer Wavelet-Transformation, das durch die Sensoren in einem Zeitbereich aufgenommene akustische Signal in die Frequenzverteilung, die Frequenz-Zeit-Verteilung, oder das Zeitsignal zu überführen, und ein der so erhaltenen Verteilungen der entsprechenden Härte des Zementklinkers zuzuordnen. Der Begriff "Wavelet-Transformation" bezeichnet dabei eine Familie von linearen Frequenz-Zeit-Transformationen. Die für die Fouriertransformation zu Grunde liegende Wertemenge im Zeitbereich ist diskret und nicht kontinuierlich vermessen, daher wird praktisch eine Diskrete Fourier Transformation durchgeführt. Der nachfolgende Code führt die Fouriertransformation über den Zeitdatenbereich. Es werden Mittelwerte aus den Fouriertransformierten bzgl. jeder einzelnen Klinkerkategorie berechnet. Das Spektrum, also die Abbildung der Amplitude in Abhängigkeit der Frequenz, wird Amplitudenspektrum genannt:

```
c=input('');
[A1,A2,A3,A4,A5,A6]=Einlesen(c);
for i=3:numel(A1)
      [f1a(:,i),g1a(:,i)]=FFT2(A1(i).A,A1(i).Tinterval);
end
F1A=mean(f1a,2, 'omitnan');
 G1A=mean(g1a,2, 'omitnan'); % Berechnung der Mittelwerte über den
Amplitudenvektor
 clear f1a g1a;
```

```
for i=3:numel(A1)
      [f1ab(:,i),g1ab(:,i)]=FFT2(A1(i).B,A1(i).Tinterval);
end
 F1AB=mean(f1ab,2,'omitnan');
 G1AB=mean(g1ab,2,'omitnan'};
  clear flab glab A1
for i=3:numel(A2)
      [f2a(:,i),g2a(:,i)]=FFT2(A2(i).A,A2(i).Tinterval);
end
F2A=mean(f2a,2,'omitnan');
G2A=mean(g2a,2,'omitnan');
clear f2a g2a
for i=3:numel(A2)
      [f2ab(:,i},g2ab(:,i)]=FFT2(A2(i),B,A2(i).Tinterval);
end
F2AB=mean(f2ab,2,'omitnan');
G2AB=mean(g2ab,2,'omitnan');
 clear A2 f2ab g2ab
for i=3:numel(A3)
      [f3a(:,i),g3a(:,i)]=FFT2(A3(i).A,A3(i).Tinterval);
end
F3A=mean(f3a,2, 'omitnan');
G3A=mean(g3a,2, 'omitnan');
clear f3a g3a
for i=3:numel(A3)
      [f3ab(:,i),g3ab(:,i)]=FFT2(A3(i).B,A3(i).Tinterval);
end
```

Die sogenannte Fast Fourier Transformation FFT dagegen nutzt Symmetrien, welche die Rechnung und damit die Rechenzeit verkürzen. Diese Art der Fouriertransformation wird beispielsweise in dem Skript FFT2.mat ausgeführt. Genutzt wird in diesem Skript die Matlab Funktion fft() für die eigentliche Transformation. Nachfolgend ist der entsprechende Code aufgelistet:

```
function [f1,Ps1,y]=FFT2(x,Y)
    y1=abs(fft(x)/length(x);        %(1) fft: Funktion Matlab,
skaliert auf Länge Zeitvektor
    fs1=1./Y;                  % fsl: Sample Rate (1/Zeitinterval)
     L1=length(y1);           % Länge Fouriertransformation
      Ps1=y1(1:L1/2+1);      % in einseitiges Spektrum umwandeln
      Ps1(2:end-1)=2*Ps1(2:end-1); % nach der Eulerschen Formel
von zweiseitigem Spektrum zu einseitigem Spektrum
      f1 = fsl*(0:L1/2)/L1;         % Skalieren der x-Achse auf
Frequenzbereich
      f1=f1';                       %Transportieren
      y=max(Ps1);                   %Maximum FFT
      Ps1=Ps1/y;                    %Normieren auf Maximum
end
```

Mittels der Frequenzverteilung im Frequenzbereich kann das akustische Signal dann einer Härte beziehungsweise einem entsprechenden Litergewicht des auf die Unterlage aufprallenden Zementklinkers zugeordnet werden. Die Zuordnung geschieht anhand bestimmter Merkmale wie beispielsweise Frequenzschwerpunkt oder Schiefe, Breite etc. der Frequenzverteilung bzw. der einzelnen Frequenzen bei der Frequenzverteilung, die charakteristisch für eine bestimmte Härte des Zementklinkers sind. Ähnlich würde die Analyse einer Wavelet-Transformation erfolgen.

In einer Ausführungsform ist daher die Auswerteeinheit zusätzlich dazu vorgesehen, die erfasste Härte des Zementklinkers einem entsprechenden Litergewicht des Zementklinkers zuzuordnen. Die Messung des Litergewichtes als Qualitätsbestimmung ist relativ alt und wurde zur Schaffung eines Vergleichsmaß für die Klinkerqualität bereits um

1936 wissenschaftlich begründet. Das Litergewicht wird durch die Betriebsgrößen der Klinkerbrennung sowie des Frei-Kalk-Gehaltes beeinflusst. Diese Zusammenhänge werden auch durch den Begriff der "Klinkerhärte" erfasst. Der Begriff "Klinkerhärte" dient dann vor allem dem generalisieren physikalischen Eigenschaften des Zementklinkers. Das Litergewicht wird gemessen, indem mittels eines Gefäßes (Litermaß) Klinkerproben entnommen werden. Durch Aufstoßen des Gefäßes auf einem festen Untergrund wird die höchste Packungsdichte des Zementklinkers im Gefäß sichergestellt und nachfolgend gewogen. Abzüglich des Gewichts des leeren Gefäßes ergibt sich das Gewicht des Zementklinkers normiert auf 1 Liter. Das Maß des Litergewichtes zeigt den Zusammenhang zum Frei-Kalk-Gehalt auf und ist damit zur Bewertung der Qualität eines Brandes geeignet. Der Begriff der Härte lässt sich durch die Betrachtung der Ursachen einer Litergewichtsveränderung hierbei vor allem auf die Porosität des Zementklinkers zurückführen, die auch an die Form des Klinkerkorn koppelt. Hier trifft im Wesentlichen die Kugelform für hohe Litergewichte zu, für niedrig litergewichtige Zementklinker sind diese unrund in der Gestalt und uneben bezogen auf die Oberfläche. Bei der Sinterung bildet sich zunächst Belit (Dicalciumsilikat), während sich Alit (Tricalciumsilikat) erst sekundär aus Belit unter weiterer Aufnahme von freiem Kalk bildet. Die Umwandlung von Belit in Alit ist abhängig von den Betriebsbedingungen des Drehofens und vorgeschalteter Komponenten der Zementherstellung. Leichter Zementklinker hat als Hauptbestandteil Belit, schwerer Zementklinker hat dagegen Alit als Hauptbestandteil, wobei Alit auch härter als Belit ist. Insofern zeigt ein Zementklinker mit hohem Litergewicht eine größere Härte als ein Zementklinker mit niedrigem Litergewicht. Da das Litergewicht eine über lange Zeit bekannte und angewendete Qualitätsmessmethode ist, ist es vorteilhaft, den sich aus dem akustischen Signal ergebenden Frequenzverteilungen, Frequenz-Zeit-Verteilungen oder Zeitsignalen Litergewichte zuzuordnen, weil hier leicht eine separate experimentelle Verifikation der Zuordnung vorgenommen werden kann, beispielsweise zur separaten Überprüfung der Korrektheit der erfolgten Zuordnungen und zum weiteren Training des verwendeten Algorithmus für die Zuordnung.

In einer weiteren Ausführungsform erfolgt die Zuordnung der Härte zur jeweilige Frequenzverteilung und/oder die Zuordnung der Härte zum Litergewichts des Zementklinkers durch die Auswerteeinheit mittels einer Abbildungsfunktion auf Basis eines maschinellen Lernens mittels entsprechender Algorithmen. Maschinelles Lernen erlaubt es, ohne explizite Kenntnis der Daten eine Systematik zu erfassen und entsprechend einer Vorhersage in vorher festgelegte Klassen zu tätigen. Die Klassen entsprechen beispielsweise Intervalle für jeweils einen anderen Bereich an Klinkerhärten. Die Abbildungsfunktion kann dabei ein Element aus der Gruppe Stützvektormaschinen, lineare Diskriminanzanalyse, logistische Regression, nächster Nachbar-Klassifikator oder neuronale Netze zur Zuordnung der Frequenzverteilung verwenden.

Eine "Stützvektormaschine" trennt die n-dimensionalen Merkmale der Frequenzverteilung mittels eines Trennvolumen, im Falle von 2 Dimensionen mittels einer Trennlinie, voneinander. Die Trennlinie sollte dabei nach Möglichkeit so in die Daten gelegt werden, dass der Abstand der zu trennenden Datenpunkte von der Trennebene möglichst groß wird, was auch als ein so genannter max. Margin Classificator bezeichnet wird. Die Stützvektoren sind in diesem Falle der Abstand zu der Trennlinie. Dieser Abstand addiert um den so genannten Bias Vektor. Mathematisch wird der Abstand zu einer Ebene minimiert. Liegen die Daten aufgrund ihrer Streubereite ineinander, sodass keine Trennebene für die Daten gefunden werden kann, kann man die Daten mittels Kernel in einen höheren Zusammenhang transformieren, sodass sich lineare Trennverfahren zu Klassifizierung verwendet werden können. Zunächst wird zugelassen, dass der Abstand zur Trennebene kleiner 1 werden kann, was in nachfolgende Nebenbedingungen resultiert:

$$w \cdot x_k + b \geq 1 - \zeta_k \qquad , y_k = 1$$

$$w \cdot x_k + b \leq -1 + \zeta_k \qquad , y_k = 2$$

mit den Stützvektoren $x_k$ und den Klassen $y_k$ = 1 und 2. Die Kernel-Funktion beschreibt hierbei den Zusammenhang in einer höheren Dimension, ohne die Daten in diese zu transferieren. Für beispielsweise 2 Klassen x und y in 2 Dimensionen:

$$k(x_j, x_k) = x_j \cdot x_k^T$$

Hier wird zudem noch der Vektor w als Linearkombination ausgeschrieben:

$$w = \sum_{yj=1} \alpha_j x_j - \sum_{yj=2} \alpha_j x_j$$

$$\sum_{y_j=1}\alpha_j k(x_j,x_k) - \sum_{y_j=2}\alpha_j k(x_j,x_k) + b \geq 1 - \zeta_k y_k = 1$$

$$\sum_{y_j=1}\alpha_j k(x_j,x_k) - \sum_{y_j=2}\alpha_j k(x_j,x_k) + b \geq -1 + \zeta_k y_k = 2$$

Dieser Ausdruck ist schließlich unter den obigen Nebenbedingungen zu optimieren:

$$min_{w,b,\zeta} = (\frac{1}{2}w \cdot w^T + C\sum_j \zeta_j)$$

C stellt hier einen Bestrafungsterm dar, welcher gegen Überanpassung wirken soll und einer reellen Zahl entspricht. Das Minimum dieses Ausdrucks kann über verschiedene Algorithmen gefunden werden. Beispielsweise stehen dafür in MathLab Lösungsalgorithmen wie "iterative Single Data Algorithmen", "Quadratische Programmierung" oder "Sequentielle Minimal-Optimierung" zur Verfügung. Vorzugsweise kann der "iterative Single Data Algorithmus" verwendet werden. Der Vorteil einer Stützvektormaschine liegt darin, dass nicht-lineare Klassengrenzen zur Klassifizierung der Daten verwendet werden können, eine Normalverteilung der Daten keine Voraussetzung ist und eine hohe Generalisierungsfähigkeit besteht. Allerdings ist die Stützvektormaschine ein aufwendiges Verfahren. Die Stützvektormaschine vermeidet zudem, durch zu viele optimierte Einstellungen ein sogenanntes Überanpassen herbeizuführen. Nachfolgende Einstellungen der Stützvektormaschine können unter den aufgelisteten Rahmenbedingungen verwendet werden:

Tabelle 1: Einstellungen der Stützvektormaschine

| **Einstellwert** | **Wirkung** |
|---|---|
| Box Constraint | Überanpassung möglich. Bestrafungsterm C zur Korrektur verwenden |
| Clip Alphas | Setzt der Wert $\alpha_j$ zu 0, wenn nahe 0. Überanpassen möglich |
| Kernel | Gauß / Linear / Polynom - keine Gefahr des Überanpassens |
| OutlierFraction | Entfernt Ausreißer in den Daten |
| Cost | Bestrafungsterm für die Fehlervorhersage einer Klasse, Angabe in Matrixform |
| Weights | Angabe der Gewichtung bestimmter Daten |
| KFold | Partitionierung der Daten in k Sätze |
| KernelScale | Skalierung der Kernel-Funktion mit einer Konstanten |

Lineare Diskriminanzanalyse: Die Diskriminanzanalyse ähnelt der Stützvektormaschine. Der Unterschied ist, dass hier kein Mindestabstand der Datenpunkte von der Ebenen eingehalten werden muss, welche die Daten aufteilt.

Logistische Regression: Die logistische Regression fittet im Gegensatz zur linearen Regression, dessen Modellunktion kontinuierliche Werte annehmen kann, eine Funktion an die Daten an, welche Werte zwischen 0 und 1 annimmt. Dieses Verfahren wird typischerweise für die binäre Klassifikation angewandt.

Nächster-Nachbar-Klassifikator: Hier werden mittles einer geeigneten Metrik die Abstände zu den nächsten Nachbardatenpunkten berechnet. Formal ist das in der nachfolgenden Gleichung festgehalten:

$$||x - x_k|| = min_{j=1...,n}||x - x_j||$$

Der Abstand zum Datenpunkt $x_j$ der Klasse j, lässt die Klassifizierung zur Klasse j zu. Im Unterschied dazu, berechnet der k-Nächste-Nachbar-Klassifikator die Zugehörigkeit aufgrund der k nächsten Nachbarn. Klassifiziert wird dann über die Anzahl der Nachbarn welche zur selben Klasse gehören. Es entscheidet die Klasse zu derer die meisten nächsten Nachbarn des Merkmals x gehören.

Neuronale Netze: Ein neuronales Netz kann für das überwachte, sowie das unüberwachte Lernen eingesetzt werden. In dieser Betrachtung wird ebenfalls lediglich das überwachte Lernen behandelt. Mathematisch ist ein Neuron

über nachfolgende Gleichung

$$v = \sum_{n=0}^{N} x_i \cdot w_i + w_0 \qquad o = \phi(v)$$

modellierbar. Die sogenannte Aktivierungsfunktion erklärt den Ausgang des Neurons in Abhängigkeit des Eingangs v und kann beispielsweise auf das Intervall [0,1] abbilden. Der Eingang v bildet sich aus obiger Gleichung aus den Eingangswerten $x_i$ und den Gewichtungen $w_i$. Der Bias ist $w_o$ und wird im Training mit den Gewichtungen angepasst. Erste Abänderungen können beispielsweise mit unterschiedlichen Aktivierungsfunktionen erzielt werden. Die Verschaltungen der einzelnen Neuronen definiert nun das neuronale Netz und damit lassen sich unterscheiden: (a) Einschichtige Netze, (b) Mehrschichtige Netze, sogenannte "Deep Learning Netze", (c) Netze mit Rückkopplung des Ausgangs, und (d) sogenannte Convolutional Neuronale Netze. Convolutional Neuronale Netzwerke erzielen die erste Aufbereitung der Daten mittels einer diskreten Faltung mit einer Faltungsmatrix. Die Faltungsmatrix ist eine Anordnung von Neuronen als Eingangsschicht in mehreren Dimensionen. Im Falle von mehreren hintereinander angeordneten Convolutional Layer (Schichten) wird dann von einem DeepConvolutional Network gesprochen. Als Schnittstelle kann das neuronale Netz die Fouriertransformation als Eingang verwenden. Für dieses Problem können alle voranstehenden Netztypen verwendet werden.

In einer Ausführungsform verwendet die Abbildungsfunktion eine Stützvektormaschine. Der Vorteil der Stützvektormaschine liegt in dem einfachen Anpassen nötiger Einstellungen und im Gegensatz beispielsweise zur Linearen Diskriminanzanalyse sind auch nicht normalverteilte Größen gut trennbar. Die Normalverteilung der Merkmale sollte nicht vorausgesetzt werden müssen. Auch das neuronale Netz ist vorteilhaft, allerdings geht dabei die physikalische Interpretierbarkeit der Daten größtenteils verloren. Außerdem muss hier ausführlich getestet werden, welcher Netztyp in Frage kommt und viele Einstellungen müssen während des Trainings berücksichtigt werden, um eine mögliche Überanpassung zu verhindern.

In einer Ausführungsform ist das maschinelle Lernen ein überwachtes Lernen unter Verwendung von Klassifizierungen und/oder Regression. Ein überwachtes Lernen ist das Trainieren des Auswertealgorithmus über Eingangs- und Ausgangsdaten, während unüberwachtes Lernen der Auswertealgorithmus nur über Eingangsdaten trainiert. Für das überwachte Lernen ist insbesondere die Stützvektormaschine vorteilhaft. Ein Überanpassung geht dabei mit einer geringen Varianz des Modells (hier die Abbildungsfunktion) in Bezug auf die Trainingsdaten einher. Auf neue Daten reagiert das Modell aber sehr schlecht, das heißt mit einer großen Varianz. Dagegen drückt sich das Unteranpassen in einer großen Varianz in den Trainingsdaten aus. Für das überwachte Lernen ist es daher sinnvoll, die Daten entsprechend in Trainingssätze und Validierungssätze zu unterteilen. Um einer Überanpassung entgegen zu wirken, ist eine angemessenen Anzahl an Trainingssätzen vorteilhaft. Es sollten dabei mehr als 10 Trainingssätze verwendet werden. Zuverlässige Ergebnisse lassen sich bereits mit bis zu 100 Trainingssätzen erzielen. In einer Ausführungsform wird daher die Abbildungsfunktion mittels einer jeweiligen Anzahl an Trainingsdatensätzen und Validierungsdatensätzen trainiert, wobei die Anzahl der Trainingsdatensätze zwischen 60% und 80% einer Gesamtanzahl an Datensätzen entspricht, vorzugsweise umfasst das Training eine Kreuzvalidierung der Datensätze umfasst. Die Gesamtanzahl der Datensätze ist hierbei die Summe der Anzahlen der Trainingsdatensätze und Validierungsdatensätze.

In einer weiteren Ausführungsform werden mittels Klassifikation Frequenzwerteintervalle zur einer Klinkerkategorie entsprechend einer bestimmten Härte des Zementklinkers zugeordnet, wobei die Klinkerkategorien eine jeweilige Breite besitzen, die so angepasst ist, dass keine Wertelücken zwischen den Klinkerkategorien verbleiben, sodass alle Frequenzverteilungswerte einer der Klinkerkategorien zugeordnet werden können. In diesem Fall kann eine größere Streubreite der Frequenzwerte toleriert werden.

In einer weiteren Ausführungsform erfolgt das Training der Abbildungsfunktion unter Produktionsbedingungen im jeweiligen Zementwerk. Damit wird die Abbildungsfunktion auf die in dem jeweiligen Zementwerk vorliegende geometrische Anordnung der Unterlage relativ zum Drehofen, insbesondere die Fallhöhe zwischen Drehofen und Unterlage und der Winkel der Unterlage relativ zur Horizontalen, trainiert. Diese geometrischen Verhältnisse beeinflussen den Aufprallschall des Zementklinkers bei derselben eigentlichen Härte, sodass in die Härtebestimmung auch die individuellen geometrischen Verhältnisse im Zementwerk eingehen und dadurch die Bestimmung der Klinkerhärte verbessert wird.

In einer weiteren Ausführungsform wird das Training zur Generierung von mehreren Merkmalen der Frequenzverteilung verwendet wird, wobei auf Basis zumindest des Merkmals mit der größten Aussagekraft die Zuordnung der erhaltenen Frequenzverteilung zu einer Klinkerkategorie ausgeführt wird. Der Begriff "Merkmal" bezeichnet eine Eigenschaft der Frequenzverteilung, nach dem sich die Frequenzverteilung klassifizieren lässt, also in eine bestimmte Kategorie (Wertebereich) für die Härte des Zementklinkers bzw. für dessen Litergewicht einordnen bzw. zuordnen lässt. Merkmale der Frequenzverteilung sind beispielsweise die Varianz oder Breite der Frequenzverteilung, die Schiefe der Frequenzverteilung oder die Wölbung der Frequenzverteilung. Diese unterschiedlichen Merkmale können eine Klassifizierung des akustischen Signals des betreffenden Zementklinkers auf die Unterlage in Klinkerhärte-Kategorien (bzw. Litergewichts-Kategorien) mit unterschiedlicher Zuverlässigkeit (Qualität) zulassen. Vorzugsweise wird die Klassifizierung nach erfolgtem Trainieren und Verifizieren des verwendeten Algorithmus anhand des am besten charakterisierenden Merkmals vorgenommen, um die zuverlässigste Zuordnung der Frequenzverteilung zu einer Härte des Zementklinkers sicherzustellen. Entsprechend kann nachfolgend dann die Abbildungsfunktion auch das Litergewicht des Zementklinkers sicherer zuzuordnen. Der nachfolgende Code generiert die Merkmale, indem nach der Fouriertransformation die Merkmale über die Funktion "moment_3" generiert werden, wobei beispielsweise X1 -X5 unter anderem den Funktionsschwerpunkt, die Varianz (Breite) der Frequenzverteilung, die Schiefe, die Wölbung der Frequenzverteilung etc. darstellen:

```
function [X1,X2,X3,X4,X5]=FFT(x,Y)
      y1=abs(fft(x)/length(x));
      fs1=1./Y;
       Ll=length(yl);
         Ps1=y1(1:L1/2+1);
         Ps1(2:end-1)=2*Ps1(2:end-1);
         f1 = fs1*(0:L1/2)/L1;
         Ps1=Ps1/max(Ps1);
[XI, X2, X3, X4, X5]=moment_3(f1,Ps1');
end
```

Ob diese Merkmale charakteristische Merkmale darstellen können und welches Merkmal davon am aussagekräftigsten ist, kann beispielsweise anhand von QQ-Plots, Tukey-Ascombe Plots, Korrelationsplots oder Boxplots überprüft werden.

In einer weiteren Ausführungsform werden als Merkmale die Schiefe oder die Breite der Frequenzverteilung verwendet, und/oder die Merkmale mittels einer einfachen gleitenden Mittelwertbildung der Frequenzverteilung mittels eines Filters geglättet beziehungsweise gefiltert werden. Der nachfolgende Algorithmus als Stützvektormaschine für die Abbildungsfunktion zeigte eine Korrelation von 96%.

```
c=input('');
if c==1
    load('F:
\Dokumentation\VorlaeufigeErgebnisse\Validierung\PAKL\Training\Dat
en_moment_1.mat);
%PAKL
else if c==2
    load('F:
\Dokumentation\VorlaeufigeErgebnisse\Validierung\PEKL\Training\Dat
en_moment_1.mat);
%PEKL
    else if c==3
    load('F:
\Dokumentation\VorlaeufigeErgebnisse\Validierung\PGKL\Training\Dat
en_moment_1.mat);
%PGKL
        else if c==4
    load('F:
\Dokumentation\VorlaeufigeErgebnisse\Validierung\PKKL\Training\Dat
en_moment_1.mat);
%PKKL
            else if c==5
\Dokumentation\VorlaeufigeErgebnisse\Validierung\SKG\Training\Date
n_moment_1.mat);
%SKG
                end
            end
```

```
            end
        end
    end

    d=input('');
    file1='SVM4.mat';
    file2='SVM4F1.mat';
    file3='SVM4F2.mat';
    file={file1,file2,file3};
    filed3='ConfusionSVM4Training.eps';
    filed2='ConfusionSVM4F1Training.eps';
    filed1='ConfusionSVM4F2Training.eps';
    filed={filed1,filed2,filed3};

    %% Trainieren der SVM
    %template SVM
    t=templateSVM('Standardize','on','KernelFunction','linear');
    %Einstellungen aus ClassificationLearner
    sz=[300 1 150];          %Fensterbreite
    a=1;           %Nenner rationale Übertragungsfunktion
    for i=1:length(sz)
    b(i)={(1/sz(i))*ones(1,sz(i))};
    end
```

Als Filter wurden hier Mittelwertfilter mit 50, 150 und 300 Werten Breite, die die gleichen guten Korrelationswerte mit der Stützvektormaschine lieferten, siehe nachfolgenden Code:

```
dm1=abs(AB(2,1)-AB(3,1));
dm2=abs(AB(3,1)-AB(1,1));
dm3=abs(AB(1,1)-AB(6,1));
dmm=min([dm1,dm2,dm3]);  % Auswahl der Intervallbreite
if dmm<100
    dmm=dmm+150;
else dmm=dmm
end
sz=[300 50 150];          %Fensterbreite
a=1;           %Nenner rationale Übertragungsfunktion
for i=1:length(sz)
```

```
b(i)={(1/sz(i))*ones(1,sz(i))};
end
clear XABf2;
for i=1:length(sz)
XABf2(i)={filter(b{1,i},a,XAB2(:,1))};
end

%ok

for i=1:length(YABr7)
if YABr7(i)=="1"
    y=y+1;
else
    y=y;
end
end
s1AB=y;
y=0;
for i=1:length(YABr7)
if YABr7(i)=="2"
    y=y+1;
else
    y=y;
end
end
s2AB=y;
y=0;
for i=1:length(YABr7)
if YABr7(i)=="3"
    y=y+1;
else
    y=y;
end
end
s3AB=y;
```

In einer weiteren Ausführungsform umfasst das maschinelle Lernen einen kontinuierlichen Vergleich zwischen dem zugeordneten Litergewicht und einem zur Verifikation in einer Messapparatur an entnommenen Proben des

Zementklinkers gemessenen Litergewichts umfasst, wobei eine eventuelle Varianz zwischen beiden Litergewichten zur kontinuierlichen Anpassung der Abbildungsfunktion verwendet wird. Durch eine kontinuierliche Verifikation der zugeordneten Litergewichte mit den tatsächlich gemessenen Litergewichten und der entsprechenden Rückkopplung der Messdaten in das Training der Abbildungsfunktion kann die Qualität der Zuordnungen der Abbildungsfunktion vom Zementklinkern zu Härten bzw. Litergewichten weiter optimiert werden.

Die Erfindung betrifft des Weiteren ein Zementwerk mit einem Drehrohrofen und einer Prozesssteuerung, mit der Prozessparameter einer Zementklinkerherstellung auf Basis einer mit der erfindungsgemäßen Vorrichtung am Auslasses des Drehofens bestimmten Härte des Zementklinkers so gesteuert werden, dass die Härte des Zementklinkers beim Verlassen des Drehrohrofens in einem gewünschten Härtebereich gehalten oder in diesen zurückgeführt werden kann. Beispielsweise können dafür zumindest eine Temperatur einer Sinterzone im Drehofen, eine Material-Verweildauer in dieser Sinterzone, der Anteil an $CaCO_3$ und/oder ein Körnungsdurchmesser eines Rohmaterials für den Zementklinker auf Basis des zugeordneten Litergewichts relativ zum gewünschten Litergewicht mittels der Prozesssteuerung variiert werden. Das Messverfahren für die experimentelle Bestimmung des Litergewichts von Proben von Zementklinker wurde bereits voranstehend beschrieben.

Damit ist es möglich, über Aufnahme der akustischen Signale die Härte des jeweiligen, gerade auf die Unterlage gefallenen Zementklinkers zu bestimmen. Da die Einflussgrößen zur Variation der Härte des Zementklinkers grundsätzlich bekannt sind, stellen diese praktisch in Echtzeit stattfindende Bestimmung der Härte des Zementklinkers eine Möglichkeit dar, sofort auf ungünstige Klinkerhärten mittels Anpassung der Produktionsparameter zu reagieren, was über die Prozesssteuerung in automatisierter Weise durchgeführt werden kann. Die Prozesssteuerung umfasst ein oder mehrere Prozessoren, Computereinheiten oder Server, auf denen ein Prozesssteuerungsprogramm installiert und ausgeführt werden kann. Das Prozesssteuerungsprogramm kann dabei beispielsweise auf Tabellen zurückgreifen, in denen die zu treffenden Maßnahmen und die entsprechend einzustellenden Prozessparameter in Abhängigkeit von der Härte des Zementklinkers bzw. des Litergewichts des Zementklinkers aufgelistet sind und entsprechend dieser Vorgaben die entsprechenden Komponenten des Zementwerks ansteuern. Diese Daten und Tabellen sind auf dafür geeigneten Datenspeichern abgelegt. Geeignete Datenspeicher sind dem Fachmann bekannt. Zur Steuerung des Zementwerks ist die Prozesssteuerung mit allen zu steuernden Komponenten mittels Datenverbindung (drahtlos oder kabelgebunden) verbunden, damit die Anpassung der Prozessparameter automatisch und unverzüglich auf Basis der Auswertungen der Härte des Zementklinkers bzw. dessen Litergewichts vorgenommen werden kann. Die Prozesssteuerung ist mit der erfindungsgemäßen Vorrichtung ebenfalls mit geeigneter Datenverbindung zumindest zur Übermittlung der bestimmten Klinkerhärte bzw. Litergewichts an die Prozesssteuerung verbunden.

Die vorliegende Erfindung betrifft des Weiteren ein Verfahren zur Bestimmung einer Härte von Zementklinker in einem Zementwerk mit der erfindungsgemäßen Vorrichtung, umfassend nachfolgende Schritte:

- Herunterfallen des einen Drehrohrofen des Zementwerks verlassenden Zementklinkers auf eine Unterlage;
- Emittieren eines für die jeweilige Härte des Zementklinkers charakteristischen akustischen Signals durch einen Aufschlag des Zementklinkers auf die Unterlage, indem die Unterlage durch den Aufschlag in Schwingungen versetzt wird, die in dem akustischen Signal resultieren;
- Aufnehmen des akustischen Signals umfassend mindestens eine für die Härte charakteristische Frequenzverteilung oder Frequenz-Zeit-Verteilung oder Zeitsignal mittels ein oder mehrerer Sensoren; und
- Zuordnen der so aufgenommenen Frequenzverteilung oder der aufgenommenen Frequenz-Zeit-Verteilung oder des aufgenommenen Zeitsignals zumindest zu der entsprechenden Härte des Zementklinkers mittels einer Auswerteeinheit, wobei die Frequenzverteilung umso breiter ist, je härter der Zementklinker ist, bzw. die Frequenz-Zeit-Verteilung eine für die Härte charakteristische Abfolge von Frequenzen über die Zeit aufweist, bzw. die Zeitverteilung einen für die Härte charakteristischen Anstieg aufweist,
- vorzugsweise überführt dabei die Auswerteeinheit mittels einer Fouriertransformation oder einer Wavelet-Transformation das akustische Signal in die Frequenzverteilung, die Frequenz-Zeit-Verteilung, oder die Zeitverteilung, um die so erhaltenen Verteilungen der entsprechenden Härte des Zementklinkers zuzuordnen.

Damit ist es möglich, über Aufnahme der akustischen Signale die Härte des jeweiligen, gerade auf die Unterlage gefallenen Zementklinkers zu bestimmen. Da die Einflussgrößen zur Variation der Härte des Zementklinkers grundsätzlich bekannt sind, stellen diese praktisch in Echtzeit stattfindende Bestimmung der Härte des Zementklinkers eine Möglichkeit dar, sofort auf ungünstige Klinkerhärten mittels Anpassung der Produktionsparameter zu reagieren, was über die Prozesssteuerung in automatisierter Weise durchgeführt werden kann.

In einer Ausführungsform des Verfahrens

- erfolgt der Schritt des Zuordnens mittels einer Abbildungsfunktion auf Basis eines maschinellen Lernens mittels entsprechender Algorithmen, vorzugsweise ordnet die Abbildungsfunktion die Härte des Zementklinkers auch einem Litergewicht des Zementklinkers zu,

und/oder

- verwendet der Schritt des Zuordnens eine Stützvektormaschine als Abbildungsfunktion, und/oder
- verwendet der Schritt des Zuordnens eine eventuelle Varianz zwischen dem zugeordneten Litergewicht und einem zur Verifikation in einer Messapparatur an entnommenen Proben des Zementklinkers gemessenen Litergewichts zur kontinuierlichen Anpassung der Abbildungsfunktion.

In einer weiteren Ausführungsform umfasst das Verfahren den weiteren Schritt:

- Steuern des Zementwerks mittels einer mit der Vorrichtung verbundenen Prozesssteuerung, die Prozessparameter einer Zementklinkerherstellung auf Basis der bestimmten Härte des Zementklinkers so anpasst, dass die Härte des Zementklinkers beim Verlassen des Drehrohrofens in einem gewünschten Härtebereich gehalten oder in diesen zurückgeführt werden kann,

Vorzugsweise werden dafür zumindest eine Temperatur einer Sinterzone im Drehofen, eine Material-Verweildauer in dieser Sinterzone, der Anteil an $CaCO_3$ und/oder ein Körnungsdurchmesser eines Rohmaterials für den Zementklinker auf Basis des zugeordneten Litergewichts relativ zum gewünschten Litergewicht mittels der Prozesssteuerung variiert.

Die vorliegende Erfindung betrifft des Weiteren einen Datenträger mit einem darauf gespeicherten Softwareprogramm zur Installation in einer Auswerteeinheit der erfindungsgemäßen Vorrichtung zumindest zur Ausführung eines Überführens und eines Zuordnens in dem erfindungsgemäßen Verfahren zur Bestimmung einer Härte von Zementklinker in einem Zementwerk.

Die voranstehend beschriebenen Ausführungsformen können vom Fachmann im Rahmen der erfindungsgemäßen Lehre beliebig miteinander auch abweichend von den Anspruchsrückbezügen kombiniert werden.

## Kurze Beschreibung der Figuren

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:

Fig.1: Schema eines Zementprozesses;

Fig.2: erfindungsgemäße Vorrichtung zur Bestimmung einer Härte von Zementklinker angeordnet am Auslass eines Drehofens eines Zementwerkes;

Fig.3: Akustisches Signal (a) als zeitliches Signal und (b) nach Fouriertransformation als Frequenzverteilung;

Fig.4: (a) schmale und (b) breite Aufprallpulse des Zementklinkers auf der Unterlage und entsprechende durch den Aufprall angeregte Frequenzverteilungen mit unterschiedlichen Schwingungsmoden der Unterlage;

Fig.5: schematischer Ablauf von der Aufnahme des akustischen Signals bis zur Zuordnung der Härte bzw. des Litergewichts des Zementklinkers;

Fig.6: schematische Darstellung von Prozessparametern mit Einfluss auf Härte und Litergewicht des Zementklinkers;

Fig.7: schematische Darstellung von maschinellem Lernen;

Fig.8: schematische Darstellung des Trainierens der Abbildungsfunktion mit Trainings- und Validierungsdaten im Betrieb des Zementwerks;

Fig.9: Frequenzschwerpunkte und Frequenzverteilungen für verschiedene Klinkerkategorien;

Fig.10: Konfusionsmatrix für die Stützvektormaschine für die korrekte Zuordnung von Härten von Zementklinker für eine Unterlage aus Stahl, wobei die Frequenzverteilungen mit und ohne Mittelwertfilter zugeordnet wurden;

Fig.11: erfindungsgemäßes Verfahren zur Bestimmung einer Härte von Zementklinker in einem Zementwerk; und

Fig.12: erfindungsgemäßer Datenträger mit darauf gespeichertem Softwareprogramm.

## Detaillierte Ausführungsbeispiele

Die hier gezeigten Ausführungsformen stellen nur Beispiele für die vorliegende Erfindung dar und dürfen daher nicht einschränkend verstanden werden. Alternative durch den Fachmann in Erwägung gezogene Ausführungsformen sind gleichermaßen vom Schutzbereich der vorliegenden Erfindung umfasst.

Fig.1 zeigt ein Schema eines Zementprozesses. Die durchgezogenen Linien bzw. Pfeile zeigen den Materiallauf, während die gestrichelten Linien die Läufe von Gasen zeigen. Kalkgestein (CaCO3) wird aus einem Steinbruch 1 abgebaut. Der Kalkstein als Rohmaterial durchläuft einen Brecher 2, in dem große Kalkbrocken aus dem Steinbruch 1 zerkleinert werden. Nach der Zerkleinerung kommt der Kalkstein in ein Mischbettsilo 3, das dazu dient, die für den späteren Sinterprozess nötigen Stoffe hinzuzufügen und das Material zu homogenisieren. Unter Homogenisieren wird die

Vergrößerung der Homogenität eines Systems, d.h. die Gleichartigkeit von Elementen und damit die Gleichheit einer physikalischen Eigenschaft über die gesamte Ausdehnung des Systems, verstanden. Anschließend wird das Material zu einem Zementwerk 20 transportiert, das die Komponenten 4 - 16 umfassen kann. Das homogenisierte Material wird dort in einer Rohmühle 4 gemahlen, weiter homogenisiert und in einem Elektrofilter 5 nachgeschalteten Silos erneut homogenisiert. In dem Elektrofilter 5 werden feine Staubanteile des Materials als auch das Abgas des Drehrohrofens 9 zur Minderung von Emissionen herausgefiltert. Das Ofenabgas durchläuft zur Kühlung vor dem Elektrofilter 5 noch einen Verdampfungskühler 6. Von den dem Elektrofilter 5 nachgeschalteten Silos aus gelangt das nun "Rohmehl" genannte Material in den sogenannten Zyklonvorwärmer 7, der das Rohmehl mit Abgasen aus dem Drehrohrofen 9 und einem Calcinator 8 auf eine Temperatur von ca. 800 °C erwärmt, um es zu entsäuern. Als Rohmehl wird das ungebrannte und homogenisierte Gemisch aus der Rohmühle bezeichnet. Unter entsäuern wird hier der Entzug von Kohlensäure verstanden, wobei der Begriff für jede Verkleinerung des Kohlensäureanteils benutzt wird. Ein Calcinator ist ein Gerät zur Entsäuerung von Kalk. Der Calcinator stellt einen gesonderten Verbrennungsraum meist in Form eines Zyklonvorwärmturms und Gegenstromwärmetauscher. Durch die Calzination wird Kalziumcarbonat zersetzt in Wasser, das Calciumoxid und das entweichende Kohlenstoffdioxid. In modernen Ofenanlagen kann durch den Einsatz eines Calcinators eine a. 90%ige Entsäuerung des Rohmehls bereits vor dem Eintritt in den Drehrohrofen erreicht werden. Nach der Entsäuerung durchläuft das Rohmehl den Drehrohrofen 9. Das Rohmehl wird in einem Drehrohrofen gesintert, wodurch der Zementklinker entsteht. Der Drehrohrohrofen 9 besitzt eine leichte Neigung von ca. 3° bis 4°. Betrieben wird der Drehrohrofen 9 von einer ca. 2000 °C heißen Flamme aus einem Brenner 10, die das Rohmehl auf ca. 1400 °C bis 1450 °C erhitzt. Bei diesen Temperaturen sintert das Rohmehl und die nachfolgende chemische Reaktion läuft ab:

$$\text{CaCO3} \xrightarrow{\text{+ Wärme}} \text{CaO + CO2}$$

Beim Sintern werden zumeist körnige oder pulvrige Stoffe vermischt und dann durch Erwärmung miteinander verbunden. Im Gegensatz zur reinen Schmelze werden hierbei jedoch keine oder zumindest nicht alle Ausgangsstoffe aufgeschmolzen. Beim Sintern macht man sich zunutze, dass Pulver eine große Oberfläche und damit eine große Oberflächenenergie besitzt, jedes System jedoch danach strebt, einen Zustand geringster freier Enthalpie einzunehmen. Beim Sintern vergrößern sich die einzelnen Körner, so dass die Oberflächenenergie sinkt. Zugleich steigt der Anteil abgesättigter chemischer Bindungen, so dass sich der Körper insgesamt verfestigt. Der Vorgang wird als Sinterprozess bezeichnet. Bei diesem Vorgang entsteht der Zementklinker. Ein Drehrohrofen ist ein zylindrischer Ofen für kontinuierliche Prozesse in der Verfahrenstechnik, der sich im Normalbetrieb kontinuierlich um die eigene Achse dreht. Verbunden mit einer leichten Neigung der Rotationsachse sorgt die Drehbewegung für den Produkt- oder Brennstofftransport. Drehrohröfen für die Zementindustrie sind in der Regel direkt beheizt, d.h. eine offene Flamme beheizt das Ofeninnere auf Temperaturen von ca. 1.450 °C. Dabei entsteht Drehrohrofenabgas, das zur Vorwärmung des Rohmehls benutzt wird. Der frisch gebrannte Zementklinker verlässt den Drehrohrofen 9 bei Temperaturen von 1.200 °C bis 1.250 °C und fällt auf einen Rostkühler 12 als Unterlage, wo er auf unter 200 °C abgekühlt wird. Die Abkühlung des Zementklinkers wird über den Rostkühler 12 als Unterlage 12 relativ schnell durchgeführt, um chemische Umwandlungsprozesse bestimmter Klinkerphasen zu vermeiden. Eine wesentliche Aufgabe des Rostkühlers 12 als Unterlage 12 ist die Wärmerückgewinnung, bei der aus einem Kühlgebläse 11 ausgebrachte Kühlluft die Wärme des heißen Zementklinkers aufnimmt, die dann im Drehrohrofen 9 als vorgewärmte Verbrennungsluft, die sogenannte Sekundärluft, oder in der Rohmühle 4 als Trockenluft eingesetzt wird. Bei dem Rostkühler 12 wird der Zementklinker auf einem Rost als Unterlage 12, das aus luftdurchlässigen Platten besteht, im Kreuzstrom zur Kühlluft transportiert und zu einem weiteren Mischbettsilo 3 weitertransportiert. In dem weiteren Mischbettsilo 3 wird der Zementklinker erneut gelagert und homogenisiert, indem Zementklinker für den Weitertransport an verschiedenen Stellen des Mischbettsilos 3 entnommen werden. Der Zementklinker werden einer Zementmühle 14 zugeführt, wo er gemeinsam mit aus einem Silo für Zuschlagstoffe 13 je nach Zementsorte zugeführten Zuschlagstoffen wie beispielsweise Gips, Trass, Hüttensand und/oder Ölschieferabbrand, zu Zement vermahlen wird. Hüttensand entsteht durch Granulation von flüssiger Hochofenschlacke mit Wasser und/oder Luft. Er ist ein feinkörniges (< 5 mm), glasiges Nebenprodukt der Roheisenherstellung im Hochofen. Hüttensand besteht aus ca. 30-45 % CaO, 30-45 % $SiO_2$, 5-15 % $Al_2O_3$, 4-17 % MgO, 0,5-1 % S und Spuren anderer Elemente. Der resultierende Zement wird über einen Sichter 15 geleitet. Unter einem Sichter wird hier eine Vorrichtung zur Klassierung von Feststoffen nach definierter Partikelgröße verstanden. Sichten ist Klassieren durch Ausnutzung der unterschiedlichen Sinkgeschwindigkeiten verschieden großer Feststoffteilchen in einem Luftstrom. Die Aufgabe des Sichters 15 ist es, zu große Partikel aus dem Mahlvorgang an den Anfang des Mahlvorgangs zu schicken. Die Partikel verbleiben im Mahlkreislauf, bis die maximale Korngröße unterschritten wird. Anschließend wird der fertige Zement in Lagersilos 16 bis zum Abtransport gelagert. In diesem Beispiel wird als Unterlage 12 ein Rostkühler verwendet. In anderen Zementwerken 20 können beispielsweise auch andere Unterlagen 12 geeignet zur Abkühlung des Zementklinkers verwendet werden.

Fig.2 zeigt die erfindungsgemäße Vorrichtung 50 zur Bestimmung einer Härte H von Zementklinker ZK angeordnet am Auslass 9a eines Drehofens 9 eines Zementwerkes 20 mit einem Drehrohrofen 9 und einer Prozesssteuerung

100, mit der Prozessparameter einer Zementklinkerherstellung auf Basis einer mit der erfindungsgemäßen Vorrichtung 50 am Auslasses 9a des Drehofens 9 bestimmten Härte H des Zementklinkers ZK so gesteuert werden, dass die Härte H des Zementklinkers ZK beim Verlassen des Drehrohrofens 9 in einem gewünschten Härtebereich gehalten oder in diesen zurückgeführt werden kann. Der Zementklinker ZK fällt dabei auf eine Unterlage 12 und versetzt durch seinen Aufschlag auf die Unterlage 12 diese in Schwingungen, was in einem für die jeweilige Härte charakteristischen akustischen Signal AS umfassend mindestens eine für die Härte charakteristische Frequenzverteilung FV oder Frequenz-Zeit-Verteilung oder Zeitsignal resultiert, wobei die Vorrichtung 50 einen oder mehrere Sensoren (60) und eine Auswerteeinheit 70 umfasst, wobei die Sensoren 60 dazu vorgesehen sind, das jeweilige akustische Signal AS aufzunehmen und die Auswerteeinheit 70 dazu vorgesehen ist, aus der aufgenommenen Frequenzverteilung FV oder der aufgenommenen Frequenz-Zeit-Verteilung oder dem aufgenommenen Zeitsignal zumindest die Härte H des Zementklinkers ZK zu bestimmen. Der oder die Sensoren 60 können dabei Klopfsensor oder Luftschallmikrofone sein.

Der Sensor 16 als Klopfsensor kann dabei abweichend von der Darstellung in Fig.2 auf der Unterlage 12 montiert oder über ein Übertragungsblech mit ihr verbunden sein. Die Detektion des Körperschalls über den Klopfsensor, beispielsweise ein Bosch Klopfsensor, kann beispielsweise über ein Pc-Oszilloskop, PicoScope3205b, ausgewertet werden. Zusätzlich oder separat zum Körperschall kann der Luftschall mittels eines Luftschallmikrofons gemessen werden, das in einer Entfernung zur Unterlage 12 angeordnet und auf diese ausgerichtet ist (siehe Fig.2), um die Stoßgeräusche sowie Schwingungen der Unterlage 12 zu detektieren. Als Luftschallmikrofon kann beispielsweise ein Kondensatormikrofon mit zusätzliche Spannungsversorgung eingesetzt werden. Da verschiedene andere Signale unabhängig vom akustischen Signal über den Luftschall das Luftschallmikroskop erreichen können, stellen Klopfsensoren die genaueren, weil spezifischeren Sensoren 60 dar. Je nach Ausführungsform der Unterlage 12 kann deren Montage aber problematisch sein, sodass Luftmikrofone in diesem Fall zu bevorzugen wären.

Fig.3 zeigt ein akustisches Signal AS (a) als zeitliches Signal und (b) nach Fouriertransformation FF als Frequenzverteilung FV. Die Auswerteeinheit 70 überführt mittels Fouriertransformation FF das durch die Sensoren 60 in einem Zeitbereich aufgenommene akustische Signal AS in die Frequenzverteilung FV, das die für die jeweilige Härte H charakteristisch ist, wodurch die so erhaltene Frequenzverteilung FV der entsprechenden Härte H des Zementklinkers ZK zugeordnet werden kann. Das zeitliche akustische Signal AS von Zementklinker ZK auf eine Unterlage 12 in Fig.3a wurde mit einer Samplerate von 100kHz, einer Aufnahmezeit von 50ms, einem Tiefpassfilter von 30kHz und einer DC-Kopplung aufgenommen. Dem zeitlichen akustischen Signal ist in Fig.3b die Frequenzverteilung FV als Amplitudensignal nach erfolgte Fouriertransformation des akustischen Signals AS aus Fig.3a gegenübergestellt.

Fig.4 zeigt (a) schmale und (b) breite Aufprallpulse des Zementklinkers ZK auf der Unterlage 12 und entsprechende durch den Aufprall angeregte akustische Signal AS mit Frequenzverteilungen FV mit unterschiedlichen Schwingungsmoden der Unterlage 12. Aus der Fallstrecke erhält der Zementklinker ZK einen Impuls. Eine Impulsänderung, bezüglich der Richtung, stellt sich dann in Folge des mechanischen Kontakts mit der Unterlage 12 ein. Die zeitliche Differentiation dieses Impulses entspricht dann einer Kraft, die auf die Unterlage 12 wirkt. Mit Annahme der Impulserhaltung, muss damit die Kraft/Zeit Kurve einer Funktion entsprechen, für die das Integral die erhaltende Größe, also in diesem Falle dem Impuls, entspricht. Stellt man die Kraft/Zeit-Funktion als Gaußkurve für eine maximale Kraft Fmax und mit einer Breite $\sigma$ dar, so kann physikalisch die Breite der Funktion mit der Härte H des Zementklinkers ZK korreliert werden. Ein weiches Material der gleichen Masse würde eine flacherer Gauß-Kurve bedeuten, entsprechend einer größeren Breite $\sigma$. Die Anregung der Unterlage 12 kann verstanden werden über die Faltung eines Eingangsimpulses mit einer entsprechenden Übertragungsfunktion, der Unterlage 12. Der Eingang des Systems ist somit eine Kraft, der Ausgang eine Verschiebung x eines Punktes der Unterlage 12. Die Verschiebung x am örtlich fixierten Punkt der Unterlage 12 drückt sich in einer entsprechenden Schwingung aus, welche aus den sich ausbildenden Wellen auf der Unterlage 12 resultiert. Diese Wellen entsprechen dem akustischen Signal AS, welcher über die Sensoren 60 entsprechend detektiert wird. Da Wellen Gebilde in Ort und Zeit sind, verändert sich die Amplitude also auch bei einem Wechsel des Betrachtungspunktes. Die Betrachtung der Schwingung findet an einem örtlich fixierten Punkt auf einer schwingenden Unterlage 12 statt. Im fixierten Punkt können nun mehrere harmonische Schwingungen vorliegen. Unterschiedlich "harte" Zementklinker-Materialien verändern die Frequenzverteilung. Ein zeitlicher schmaler Kraft-Impuls sorgt für eine breite Anregung der Schwingungsmoden der Unterlage 12, auch der der höheren Schwingungsmoden. Ein zeitlich breiter Kraft-Impuls entspricht einem im Frequenzraum schmalen Puls und damit einer schwachen Anregung höherer Schwingungsmoden (stark abfallende Amplitudenhöhe) in der Frequenzverteilung FV, siehe Fig.4b. Ein harter Zementklinker ZK resultiert in einem ins Verhältnis zu einem weichen Zementklinker ZK gesetzten, schmaleren Impuls im Zeitbereich. Ein schmaler Impuls im Zeitbereich regt eine breitere und ausgeprägtere Frequenzverteilung FV mit vielen Frequenzen hoher Amplitude an als ein zeitlich breiterer Impuls, siehe Fig.4a.

Fig.5 zeigt einen schematischen Ablauf von der Aufnahme des akustischen Signals AS bis zur Zuordnung der Härte H bzw. des Litergewichts LG des Zementklinkers ZK. Hierbei wird eine Fouriertransformation FF oder eine Wavelet-Transformation WLT zur Übertragung des akustischen Signals AS in ein Frequenzverteilung FV, in eine Frequenz-Zeit-Verteilung FZV, oder in ein Zeitsignal ZV verwendet. Dieser Übertragung kann in einer Ausführungsform eine Filter 90 vorgeschaltet sein. Das Training der Abbildungsfunktion ABF kann auch zur Generierung von mehreren Merkmalen MM

der Frequenzverteilung FV verwendet werden, wobei auf Basis zumindest des Merkmals MM mit der größten Aussagekraft die Zuordnung der erhaltenen Frequenzverteilung FV zu einer Klinkerkategorie (Härte H, Litergewicht LG) ausgeführt wird. Die Merkmale MM können dabei die Schiefe oder die Breite der Frequenzverteilung FV sein. Die Merkmale MM können auch mittels einer einfachen gleitenden Mittelwertbildung der Frequenzverteilung FV mittels des Filters 90 geglättet beziehungsweise gefiltert werden. Als analoge Filter 90 stehen klassisch Tiefpass, Hochpass oder Bandpassfilter zur Auswahl. Auch digital, lassen sich diese Filtertypen realisieren. FIR Filter 90 sind digitale Filter mit endlicher Impulsantwort, dazu fällt auch die einfache Ausführung des gleitenden Mittelwertes. Der Filter 90 ist stabil und kann selbstständig keine Schwingung ausführen.

Fig.6 zeigt eine schematische Darstellung von Prozessparametern mit Einfluss auf Härte H und Litergewicht LG des Zementklinkers ZK. Auf das Litergewicht haben die Verrundung des Zementklinkers ZK, die Verringerung der Porosität der Klinkergranalien und damit die Vergrößerung des Raumgewichtes der Klinkergranalien Einfluss. Der Anteil des freien Kalks im Zementklinker ZK kann an einem zu hohen Kalkgehalt im Rohmehl , einem zu grob gemahlenen Rohmehl, einer ungenügenden Homogenisierung des Rohmehl, einer zu niedrigen Brenntemperatur, einer zu langsamen Klinkerkühlung , einer ungleichmäßigen Aufnahme der Brennstoffasche liegen. Die Verrundung des Zementklinkers ZK resultiert in einer höheren Packungsdichte des geschütteten Materials und damit des Litergewichts. Eine Abweichung der Kugelform lässt damit das Volumen zwischen den Klinkerkörnern anwachsen und das Litergewicht geringer werden. Zurückgeführt wird dieser Effekt auf die Durchwälzung des Materials in der Sinterzone und ist damit dem der Aufenthaltszeit in der Sinterzone t zuzuschreiben. Eine Verrundung resultiert ebenfalls in einer weniger porösen bzw. glatteren Oberfläche. Um eine Litergewichtsveränderung zusätzlich der Porigkeit zuzuschreiben, folgt die Betrachtung des spezifischen Gewichtes des Zementklinkers ZK. Dieses ändert sich nach dem Austreiben von CO2, also nach dem Kalzinator nicht mehr, es bleibt annähernd konstant bei einem Gewicht von 3,22 kg/L. Es ist daher davon auszugehen, dass der Einfluss der Litergewichtsänderung vor allem aus den Vorgängen in der Sinterzone des Drehrohrofens bestimmt wird, die sich in einer gearteten Weise auf das Raumgewicht bzw. der Porosität auswirken. Die Korrelation des freien Kalkgehaltes zum Litergewicht ist ebenfalls in der Art gegeben, dass mit steigendem Litergewicht der Gehalt an freiem Kalk (CaO) abnimmt. Daher geht die Litergewichtsveränderung auch mit der Porosität einher. Ein weiterer das Litergewicht beeinflussender Vorgang ist die Bildung des Alit aus Belit. Alit bildet sich erst sekundär aus Belit, unter Aufnahme von weiterem freien Kalk. Diese Reaktion, welche im hinteren Bereich des Drehrohrofens bei Temperaturen um T = 1250 °C abläuft, ist förderlich durch eine knetende Wirkung des Materials sowie einer flüssigen Phase. Die flüssige Phase ist vor allem auf das Vorhandensein von genug Aluminaten und Ferriten zurückzuführen. Ein Übergang von kleinen zu großen Litergewichten bedingt sich in einer Zunahme in Anzahl und Größe der Alitkristalle, hingegen in einer Größenabnahme der oft rundlich erscheinenden Belitkristalle. Der beschriebene Vorgang läuft damit unter unzureichenden Bedingungen nicht zu Gunsten eines korrekten Verhältnisses aus Belit zu Alit ab. Die Folge können Clusterbildung von freiem Kalk sein oder eben die hierzu beobachtete Porosität des Zementklinker ZK, welche auf eine geringe flüssige Phase zurückzuführen ist. Eine weitere Eigenschaft des Zementklinkers ZK mit dem Litergewicht ist die Mahlbarkeit. Der Mahlwiderstand, welcher über Siebrückstände in der Arbeit definiert wird, nimmt für die Litergewichte zu. Im Falle eines schlecht gebrannten Zementklinker ZK ist viel verstreuter Frei-Kalk aufzufinden. Somit können die Temperatur T der Sinterzone im Drehofen, die Material-Verweildauer t in dieser Sinterzone, der Anteil AK an $CaCO_3$ und/oder der Körnungsdurchmesser KD eines Rohmaterials für den Zementklinker ZK das Litergewichts LG stark beeinflussen und eignen sich daher besonders als Steuerparameter für eine Prozesssteuerung des Brennprozesses zur Herstellung von Zementklinker ZK, um das Litergewicht beziehungsweise die Härte H des Zementklinkers ZK im gewünschten Bereich zu halten oder in diesen nach einer starken Abweichung wieder zurückzuführen.

Fig.7 zeigt eine schematische Darstellung von maschinellem Lernen ML. Beim maschinellen Lernen ML unterscheidet man zwischen dem überwachten ÜL und dem unüberwachten Lernen NL. Überwacht bedeutet in dem Kontext, dass das Ergebnis der Klassifizierung vorgegeben wird (das Litergewicht). Die Aufgabe ist nun, einer Klinkerprobe eine bestimmte Härte und darauf basierend ggf. auch dem richtigen Litergewicht zuzuordnen. Damit liegt das Problem in der Klassifizierung der Datensätze. Weitere Möglichkeiten des maschinellen Lernens sind die Regression und das Clustern. Regression entspricht einer Generierung eines statistischen Modells, beispielsweise einer Vorhersage auf Grundlage eines an die Daten gefitteten Funktionsverlauf. Das Clustern ist in die Kategorie des unüberwachten Lernens einzuordnen, in welchem keinerlei Information zu den Datensätzen vorhanden ist. Beim Clustern steht also die Trennung der Daten im Vordergrund. Das maschinelle Lernen ML als überwachtes Lernen UL unter Verwendung von Klassifizierungen K und/oder Regression R ist hierbei bevorzugt. Mittels Klassifikation K werden dabei Frequenzwerteintervalle zur einer Klinkerkategorie KK entsprechend einer bestimmten Härte H des Zementklinkers ZK zugeordnet, wobei die Klinkerkategorien KK eine jeweilige Breite besitzen, die vorzugsweise so angepasst ist, dass keine Wertelücken zwischen den Klinkerkategorien KK verbleiben, sodass alle Frequenzverteilungswerte einer der Klinkerkategorien KK zugeordnet werden können.

Fig.8 zeigt schematische Darstellung des Trainierens der Abbildungsfunktion ABF mit Trainings- und Validierungsdaten TD, VD beim Betrieb des Zementwerks 20. Die Zuordnung der Härte H zur jeweilige Frequenzverteilung FV und/oder die Zuordnung der Härte H zum Litergewichts LG des Zementklinkers ZK erfolgt durch die Auswerteeinheit 70

mittels einer Abbildungsfunktion ABF auf Basis eines maschinellen Lernens ML mittels entsprechender Algorithmen, hier eine Stützvektormaschine SVM. Die Abbildungsfunktion ABF wird dabei mittels einer jeweiligen Anzahl an Trainingsdatensätzen TD und Validierungsdatensätzen (VD) trainiert wird, wobei die Anzahl der Trainingsdatensätze TD zwischen 60% und 80% einer Gesamtanzahl an Datensätzen TD, VD entspricht, vorzugsweise umfasst das Training eine Kreuzvalidierung der Datensätze TD, VD umfasst. Das Training der Abbildungsfunktion ABF erfolgt hierbei unter Produktionsbedingungen im jeweiligen Zementwerk 20. In dieser Ausführungsform umfasst das maschinelle Lernen ML auch einen kontinuierlichen Vergleich zwischen dem zugeordnetem Litergewicht LG und einem zur Verifikation in einer Messapparatur 80 an entnommenen Proben des Zementklinkers ZK gemessenen Litergewichts LG', wobei eine eventuelle Varianz zwischen beiden Litergewichten LG, LG' zur kontinuierlichen Anpassung der Abbildungsfunktion ABF und damit zur Qualitätssteigerung bei der Klassifizierung der Frequenzverteilung FV zur Echtzeit-Bestimmung von Härte H und Litergewicht LG des gerade produzierten Zementklinkers ZK verwendet wird.

Fig.9 zeigt Frequenzschwerpunkte und Frequenzverteilungen FV für verschiedene Klinkerkategorien KK. Hier wurde vier Klinkerkategorien KK von Zementklinker ZK untersucht. Kategorie 1 hat ein Litergewicht von 1,477 kg, Kategorie 2 hat ein Litergewicht von 1,54 kg, Kategorie 3 hat ein Litergewicht von 1,65 kg und Kategorie 4 hat ein Litergewicht von 1,73 kg. Mit steigendem Litergewicht steigt einerseits der Frequenzschwerpunkt an. Für diese Litergewichte wurde in Fig.10 die Konfusionsmatrizen erhalten.

Fig.10 zeigt Konfusionsmatrizen für die Stützvektormaschine für die korrekte Zuordnung von Härten H von Zementklinker ZK für eine Unterlage 12 aus Stahl, wobei die Frequenzverteilungen FV mit und ohne Filter 90 hier als Mittelwertfilter zugeordnet wurden. Zeilen der Konfusionsmatrix bezeichnen die Richtigen Klinkerkategorien (Klassen), die Spalten die Vorhersagen zu einer Klinkerkategorie (Klasse). Die Werte zeigen dann immer die Anzahl der Einordnungen in das jeweilige Feld an. Somit kann nicht nur betrachtet werden, ob eine Klinkerkategorie (Klasse) richtig oder falsch eingeordnet wurde, sondern auch in welche Klinkerkategorie (Klasse) anstelle der richtigen Klinkerkategorie (Klasse) eingestuft wurde. Die Summe über alle Felder der Tabelle gibt die Gesamtzahl der Vorhersagen bzw. die Anzahl der Datenpunkte des Datensatzes. An den Rändern der Konfusionsmatrix werden die Quotienten aus Richtigen Vorhersagen zu falschen Vorhersagen, einer Spalte bzw. einer Zeile, eingetragen. Die Vorhersagen können unterteilt werden in eine richtig vorhergesagte Klinkerkategorien, aber auch in eine richtig abgelehnte Klinkerkategorie. Die Richtig Positive Rate wird auch als Sensitivität bezeichnet, die Richtig Negative Rate auch als Spezifizität. Eine hohe Trennschärfe drückt sich demnach in einer hohen Richtig positiven Rate sowie einer hohen Richtig negativen Rate aus. Der Begriff der Genauigkeit der Vorhersagen wird in dieser Arbeit stets dem Wert zugeschrieben:

$$\frac{\sum_i TP_i}{\sum TP_i+\sum TN_i+\sum FP_i+\sum FN_i}$$

Hiermit wird die Gesamtanzahl der Richtigen Vorhersagen durch die Gesamtanzahl aller Vorhersagen dividiert. TP entspricht der Rate der richtig vorhergesagten Klinkerkategorie, FP der Rate der falsch vorhergesagten Klinkerkategorie, TN der Rate der richtig abgelehnten Klinkerkategorie und FN der Rate der falsch abgelehnten Klinkerkategorie. Fig.10 zeigt, dass eine Bestimmung der Härte H bzw. des Litergewichts LG der Klinkerproben über die Abbildungsfunktion ABS möglich ist. Die Klinkerkategorien wurden mit einer Wahrscheinlichkeit von 96% richtig eingeordnet. Mit der Verwendung der Stützvektormaschine SVM als Abbildungsfunktion ABS tragen alle Merkmale MM zum Erfolg des Verfahrens bei. Am ausgeglichensten wird das Ergebnis, wenn zusätzlich die Merkmale MM mittels einer einfachen gleitenden Mittelwertbildung geglättet respektive gefiltert werden. Als Filterbreiten wurden hier 50 Werte, 150 Werte und 300 Werte getestet. Für alle Filterbreiten ergab sich das gleiche zuverlässige Ergebnis. Erst ab Filterbreiten von weniger als 10 Werten nahm die Wahrscheinlichkeit der richtigen Klassifizierung deutlich ab.

Fig.11 zeigt ein erfindungsgemäßes Verfahren 200 zur Bestimmung einer Härte H von Zementklinker ZK in einem Zementwerk 20 mit der erfindungsgemäßen Vorrichtung 50 umfassend nachfolgende Schritte des Herunterfallens 210 des einen Drehrohrofen 9 des Zementwerks 20 verlassenden Zementklinkers ZK auf eine Unterlage 12, des Emittierens 220 eines für die jeweilige Härte des Zementklinkers charakteristischen akustischen Signals AS durch einen Aufschlag des Zementklinkers ZK auf die Unterlage 12, indem die Unterlage 12 durch den Aufschlag in Schwingungen versetzt wird, die in dem akustischen Signal AS resultieren; des Aufnehmens 230 des akustischen Signals AS umfassend mindestens eine für die Härte charakteristische Frequenzverteilung FV oder Frequenz-Zeit-Verteilung oder Zeitsignal mittels ein oder mehrerer Sensoren 60; und des Zuordnens 250 der so aufgenommenen Frequenzverteilung FV oder der aufgenommenen Frequenz-Zeit-Verteilung oder des aufgenommenen Zeitsignals zumindest zu der entsprechenden Härte H des Zementklinkers ZK mittels einer Auswerteeinheit 70, wobei die Frequenzverteilung FV umso breiter ist, je härter der Zementklinker ZK ist, bzw. die Frequenz-Zeit-Verteilung FZV eine für die Härte charakteristische Abfolge von Frequenzen über die Zeit aufweist, bzw. die Zeitverteilung ZV einen für die Härte charakteristischen Anstieg aufweist. Hierbei kann die Auswerteeinheit 70 mittels einer Fouriertransformation FF oder einer Wavelet-Transformation WLT das akustische Signal AS in die Frequenzverteilung FV, die Frequenz-Zeit-Verteilung FZV, oder die Zeitverteilung ZV

überführen 240, um die so erhaltenen Verteilungen der entsprechenden Härte H des Zementklinkers ZK zuzuordnen 250. Hierbei kann der Schritt des Zuordnens 250 mittels einer Abbildungsfunktion ABF auf Basis eines maschinellen Lernens mittels entsprechender Algorithmen erfolgen, vorzugsweise ordnet die Abbildungsfunktion ABF die Härte H des Zementklinkers ZK auch einem Litergewicht LG des Zementklinkers ZK zu, und/oder es wird eine Stützvektormaschine SVM als Abbildungsfunktion ABF verwendet, und/oder es wird eine eventuelle Varianz zwischen dem zugeordneten Litergewicht LG und einem zur Verifikation in einer Messapparatur 80 an entnommenen Proben des Zementklinkers ZK gemessenen Litergewichts LG' zur kontinuierlichen Anpassung der Abbildungsfunktion ABF verwendet. Das Verfahren kann zusätzlich den weiteren Schritt des Steuerns 260 des Zementwerks mittels einer mit der Vorrichtung 50 verbundenen Prozesssteuerung 100 umfassen, die Prozessparameter einer Zementklinkerherstellung auf Basis der bestimmten Härte H des Zementklinkers ZK so anpasst, dass die Härte H des Zementklinkers ZK beim Verlassen des Drehrohrofens 9 in einem gewünschten Härtebereich gehalten oder in diesen zurückgeführt werden kann. Für die Prozesssteuerung können Parameter wie eine Temperatur T einer Sinterzone im Drehofen, eine Material-Verweildauer t in dieser Sinterzone, der Anteil AK an $CaCO_3$ und/oder ein Körnungsdurchmesser KD eines Rohmaterials für den Zementklinker ZK auf Basis des zugeordneten Litergewichts LG relativ zum gewünschten Litergewicht mittels der Prozesssteuerung 100 variiert werden.

Fig.12 zeigt einen erfindungsgemäßen Datenträger 300 mit darauf gespeichertem Softwareprogramm 310 zur Installation in einer Auswerteeinheit einer erfindungsgemäßen Vorrichtung 50 zumindest zur Ausführung eines Überführens 240 und eines Zuordnens 250 in einem erfindungsgemäßen Verfahren 200 zur Bestimmung einer Härte H von Zementklinker ZK in einem Zementwerk 20.

**Bezugszeichenliste**:

| | |
|---|---|
| 1 | Steinbruch |
| 2 | Brecher |
| 3 | Mischbettsilo |
| 4 | Rohmühle |
| 5 | Elektrofilter |
| 6 | Verdampfungskühler |
| 7 | Zyklonvorwämer |
| 8 | Calcinator |
| 9 | Drehrohrofen |
| 9a | Auslass des Drehofens |
| 10 | Brenner |
| 11 | Kühlgebläse |
| 12 | Rostkühler beziehungsweise Unterlage |
| 13 | Silos für Zuschlagstoffe |
| 14 | Zementmühle |
| 15 | Sichter |
| 16 | Lagersilo |
| 20 | Zementwerk, erfindungsgemäßes Zementwerk |

| | |
|---|---|
| 50 | erfindungsgemäße Vorrichtung zur Bestimmung einer Härte von Zementklinker |
| 60 | Sensor zur Aufnahme des jeweiligen Akustisches Signals, vorzugsweise ein Klopfsensor |
| 70 | Auswerteeinheit zur Zuordnung des detektierten akustischen Signals zumindest zu einer Härte des, ggf. zusätzliche Zuordnung zu einem Litergewicht |
| 80 | Messapparatur zur Messung des Litergewichts an entnommenen Proben des Zementklinkers |
| 90 | Filter für die Bearbeitung des Akustisches Signals |
| 100 | Prozesssteuerung des Zementwerks |

| | |
|---|---|
| 200 | erfindungsgemäßes Verfahren zur Bestimmung einer Härte von Zementklinker in einem Zementwerk |
| 210 | Herunterfallen des Zementklinkers auf eine Unterlage |
| 220 | Emittieren eines akustischen Akustisches Signals durch einen Aufschlag des Zementklinkers auf die Unterlage |
| 230 | Aufnehmen des Akustisches Signals mittels ein oder mehrerer Sensoren |
| 240 | Überführen des Akustisches Signal mittels Fouriertransformation in einen Frequenzbereich |
| 250 | Zuordnen der Frequenzverteilung zumindest zu der entsprechenden Härte des Zementklinkers mittels einer Auswerteeinheit, ggf. auch zu einem Litergewicht des Zementklinkers |
| 260 | Steuern des Zementwerks mittels einer mit der erfindungsgemäßen Vorrichtung verbundenen Prozesssteue- |

rung auf Basis der bestimmten Härte des Zementklinkers

300 Datenträger
310 Softwareprogramm

ABF Abbildungsfunktion
AK Anteil an $CaCO_3$
AS Aufprallschall
FF Fouriertransformation
FV Frequenzverteilung
FZV Frequenz-Zeit-Verteilung
H Härte
K Klassifikation
KK Klinkerkategorie
KD Körnungsdurchmesser eines Rohmaterials für den Zementklinker
LG durch die Vorrichtung zugeordnetes Litergewicht des Zementklinkers
LG' durch die Messapparatur gemessenes Litergewicht des Zementklinkers
ML maschinelles Lernen
MM Merkmale der Frequenzverteilung
NL nicht-überwachtes Lernen
R Regression
SVM Stützvektormaschine
T Temperatur einer Sinterzone im Drehofen
t Material-Verweildauer in der Sinterzone
TD Trainingsdatensätze
ÜL überwachtes Lernen
VD Validierungsdatensätze
WLT Wavelet-Transformation
ZK Zementklinker
ZV Zeitverteilung

**Patentansprüche**

1. Eine Vorrichtung (50) zur Bestimmung einer Härte (H) von Zementklinker (ZK) in einem Zementwerk (20), wobei der Zementklinker (ZK), der einen Drehrohrofen (9) des Zementwerks (20) verlässt, auf eine Unterlage (12) fällt und durch einen Aufschlag auf die Unterlage (12) diese in Schwingungen versetzt, was in einem für die jeweilige Härte charakteristischen akustischen Signal (AS) umfassend mindestens eine für die Härte charakteristische Frequenzverteilung (FV) oder Frequenz-Zeit-Verteilung (FZV) oder Zeitsignal (ZV) resultiert, wobei die Vorrichtung (50) einen oder mehrere Sensoren (60) und eine Auswerteeinheit (70) umfasst, wobei die Sensoren (60) dazu vorgesehen sind, das jeweilige akustische Signal (AS) aufzunehmen und die Auswerteeinheit (70) dazu vorgesehen ist, aus der aufgenommenen Frequenzverteilung (FV) oder der aufgenommenen Frequenz-Zeit-Verteilung oder dem aufgenommenen Zeitsignal zumindest die Härte (H) des Zementklinkers (ZK) zu bestimmen,
vorzugsweise sind der oder die Sensoren (60) ein oder mehrere Elemente aus der Gruppe Klopfsensor oder Luftschallmikrofon, besonders bevorzugt sind der oder die Sensoren (60) Klopfsensoren.

2. Die Vorrichtung (50) nach Anspruch 1,
**dadurch gekennzeichnet,**
**dass** die Frequenzverteilung (FV) umso breiter ist, je härter der Zementklinker (ZK) ist, bzw. die Frequenz-Zeit-Verteilung eine für die Härte charakteristische Abfolge von Frequenzen über die Zeit aufweist, bzw. die Zeitsignal einen für die Härte charakteristischen Anstieg aufweist.

3. Die Vorrichtung (50) nach Anspruch 1 oder 2,
**dadurch gekennzeichnet,**
**dass** die Auswerteeinheit (70) dazu vorgesehen ist, zum Beispiel mittels einer Fouriertransformation (FF) oder einer Wavelet-Transformation, das durch die Sensoren (60) in einem Zeitbereich aufgenommene akustische Signal (AS) in die Frequenzverteilung (FV), die Frequenz-Zeit-Verteilung (FZV), oder das Zeitsignal (ZV) zu überführen, und ein der so erhaltenen Verteilungen der entsprechenden Härte (H) des Zementklinkers (ZK) zuzuordnen.

4. Die Vorrichtung (50) nach Anspruch einem der voranstehenden Ansprüche,
**dadurch gekennzeichnet,**
**dass** die Auswerteeinheit (70) zusätzlich dazu vorgesehen ist, die erfasste Härte (H) des Zementklinkers (ZK) einem entsprechenden Litergewicht (LG) des Zementklinkers (ZK) zuzuordnen.

5. Die Vorrichtung (50) nach Anspruch 4,
**dadurch gekennzeichnet,**

**dass** die Zuordnung der Härte (H) zur jeweilige Frequenzverteilung (FV) oder Frequenz-Zeit-Verteilung (FZV) oder Zeitsignal (ZV) und/oder die Zuordnung der Härte (H) zum Litergewichts (LG) des Zementklinkers (ZK) durch die Auswerteeinheit (70) mittels einer Abbildungsfunktion (ABF) auf Basis eines maschinellen Lernens (ML) mittels entsprechender Algorithmen erfolgt,
vorzugsweise ist das maschinelle Lernen (ML) ein überwachtes Lernen (UL) unter Verwendung von Klassifizierungen (K) und/oder Regression (R).

6. Die Vorrichtung (50) nach Anspruch 5,
**dadurch gekennzeichnet,**
**dass** bei der Frequenzverteilung (FV) mittels Klassifikation (K) Frequenzwerteintervalle zur einer Klinkerkategorie (KK) entsprechend einer bestimmten Härte (H) des Zementklinkers (ZK) zugeordnet werden, wobei die Klinkerkategorien (KK) eine jeweilige Breite besitzen, die so angepasst ist, dass keine Wertelücken zwischen den Klinkerkategorien (KK) verbleiben, sodass alle Frequenzverteilungswerte einer der Klinkerkategorien (KK) zugeordnet werden können.

7. Die Vorrichtung (50) nach Anspruch 5 oder 6,
**dadurch gekennzeichnet,**
**dass** die Abbildungsfunktion (ABF) eine Stützvektormaschine (SVM) verwendet.

8. Die Vorrichtung (50) nach einem der Ansprüche 5 bis 7,
**dadurch gekennzeichnet,**
**dass** die Abbildungsfunktion (ABF) mittels einer jeweiligen Anzahl an Trainingsdatensätzen (TD) und Validierungsdatensätzen (VD) trainiert wird, wobei die Anzahl der Trainingsdatensätze (TD) zwischen 60% und 80% einer Gesamtanzahl an Datensätzen (TD, VD) entspricht, vorzugsweise umfasst das Training eine Kreuzvalidierung der Datensätze (TD, VD) umfasst.

9. Die Vorrichtung (50) nach einem der Ansprüche 5 bis 8,
**dadurch gekennzeichnet,**
**dass** das Training der Abbildungsfunktion (ABF) unter Produktionsbedingungen im jeweiligen Zementwerk (20) erfolgt.

10. Die Vorrichtung (50) nach einem der Ansprüche 8 oder 9,
**dadurch gekennzeichnet,**
**dass** das Training zur Generierung von mehreren Merkmalen (MM) der Frequenzverteilung (FV) verwendet wird, wobei auf Basis zumindest des Merkmals (MM) mit der größten Aussagekraft die Zuordnung der erhaltenen Frequenzverteilung (FV) zu einer Klinkerkategorie ausgeführt wird.

11. Die Vorrichtung (50) nach Anspruch 10,
**dadurch gekennzeichnet,**

**dass** als Merkmale (MM) eine Schiefe oder eine Breite der Frequenzverteilung (FV) verwendet werden, und/oder
die Merkmale (MM) mittels einer einfachen gleitenden Mittelwertbildung der Frequenzverteilung (FV) mittels eines Filters (90) geglättet beziehungsweise gefiltert werden.

12. Die Vorrichtung (50) nach einem der Ansprüche 5 bis 11,
**dadurch gekennzeichnet,**
**dass** das maschinelle Lernen einen kontinuierlichen Vergleich zwischen dem zugeordnetem Litergewicht (LG) und einem zur Verifikation in einer Messapparatur (80) an entnommenen Proben des Zementklinkers (ZK) gemessenen Litergewichts (LG') umfasst, wobei eine eventuelle Varianz zwischen beiden Litergewichten (LG, LG') zur kontinuierlichen Anpassung der Abbildungsfunktion (ABF) verwendet wird.

**13.** Ein Zementwerk (20) mit einem Drehrohrofen (9) und einer Prozesssteuerung (100), mit der Prozessparameter einer Zementklinkerherstellung auf Basis einer mit einer Vorrichtung (50) nach einem der voranstehenden Ansprüche am Auslasses (9a) des Drehofens (9) bestimmten Härte (H) des Zementklinkers (ZK) so gesteuert werden, dass die Härte (H) des Zementklinkers (ZK) beim Verlassen des Drehrohrofens (9) in einem gewünschten Härtebereich gehalten oder in diesen zurückgeführt werden kann, vorzugsweise werden dafür zumindest eine Temperatur (T) einer Sinterzone im Drehofen, eine Material-Verweildauer (t) in dieser Sinterzone, der Anteil (AK) an $CaCO_3$ und/oder ein Körnungsdurchmesser (KD) eines Rohmaterials für den Zementklinker (ZK) auf Basis des zugeordneten Litergewichts (LG) relativ zum gewünschten Litergewicht mittels der Prozesssteuerung (100) variiert.

**14.** Ein Verfahren (200) zur Bestimmung einer Härte (H) von Zementklinker (ZK) in einem Zementwerk (20) mit einer Vorrichtung (50) nach einem der Ansprüche 1 bis 12, umfassend nachfolgende Schritte

- Herunterfallen (210) des einen Drehrohrofen (9) des Zementwerks (20) verlassenden Zementklinkers (ZK) auf eine Unterlage (12);
- Emittieren (220) eines für die jeweilige Härte des Zementklinkers charakteristischen akustischen Signals (AS) durch einen Aufschlag des Zementklinkers (ZK) auf die Unterlage (12), indem die Unterlage (12) durch den Aufschlag in Schwingungen versetzt wird, die in dem akustischen Signal (AS) resultieren;
- Aufnehmen (230) des akustischen Signals (AS) umfassend mindestens eine für die Härte charakteristische Frequenzverteilung (FV) oder Frequenz-Zeit-Verteilung (FZV) oder Zeitsignal (ZV) mittels ein oder mehrerer Sensoren (60); und
- Zuordnen (250) der so aufgenommenen Frequenzverteilung (FV) oder der aufgenommenen Frequenz-Zeit-Verteilung (FZV) oder des aufgenommenen Zeitsignals (ZV) zumindest zu der entsprechenden Härte (H) des Zementklinkers (ZK) mittels einer Auswerteeinheit (70), wobei die Frequenzverteilung (FV) umso breiter ist, je härter der Zementklinker (ZK) ist, bzw. die Frequenz-Zeit-Verteilung (FZV) eine für die Härte charakteristische Abfolge von Frequenzen über die Zeit aufweist, bzw. die Zeitverteilung (ZV) einen für die Härte charakteristischen Anstieg aufweist,
- vorzugsweise überführt (240) dabei die Auswerteeinheit (70) mittels einer Fouriertransformation (FF) oder einer Wavelet-Transformation (WLT) das akustische Signal (AS) in die Frequenzverteilung (FV), die Frequenz-Zeit-Verteilung (FZV), oder die Zeitverteilung (ZV), um die so erhaltenen Verteilungen der entsprechenden Härte (H) des Zementklinkers (ZK) zuzuordnen.

**15.** Das Verfahren (200) nach Anspruch 14, wobei der Schritt des Zuordnens (250)

- mittels einer Abbildungsfunktion (ABF) auf Basis eines maschinellen Lernens mittels entsprechender Algorithmen erfolgt, vorzugsweise ordnet die Abbildungsfunktion (ABF) die Härte (H) des Zementklinkers (ZK) auch einem Litergewicht (LG) des Zementklinkers (ZK) zu,
und/oder
- eine Stützvektormaschine (SVM) als Abbildungsfunktion (ABF) verwendet, und/oder
- eine eventuelle Varianz zwischen dem zugeordneten Litergewicht (LG) und einem zur Verifikation in einer Messapparatur (80) an entnommenen Proben des Zementklinkers (ZK) gemessenen Litergewichts (LG') zur kontinuierlichen Anpassung der Abbildungsfunktion (ABF) verwendet.

**16.** Das Verfahren (200) nach Anspruch 14 oder 15, umfassend den weiteren Schritt:

- Steuern (260) des Zementwerks mittels einer mit der Vorrichtung (50) verbundenen Prozesssteuerung (100), die Prozessparameter einer Zementklinkerherstellung auf Basis der bestimmten Härte (H) des Zementklinkers (ZK) so anpasst, dass die Härte (H) des Zementklinkers (ZK) beim Verlassen des Drehrohrofens (9) in einem gewünschten Härtebereich gehalten oder in diesen zurückgeführt werden kann,
- vorzugsweise werden dafür zumindest eine Temperatur (T) einer Sinterzone im Drehofen, eine Material-Verweildauer (t) in dieser Sinterzone, der Anteil (AK) an $CaCO_3$ und/oder ein Körnungsdurchmesser (KD) eines Rohmaterials für den Zementklinker (ZK) auf Basis des zugeordneten Litergewichts (LG) relativ zum gewünschten Litergewicht mittels der Prozesssteuerung (100) variiert.

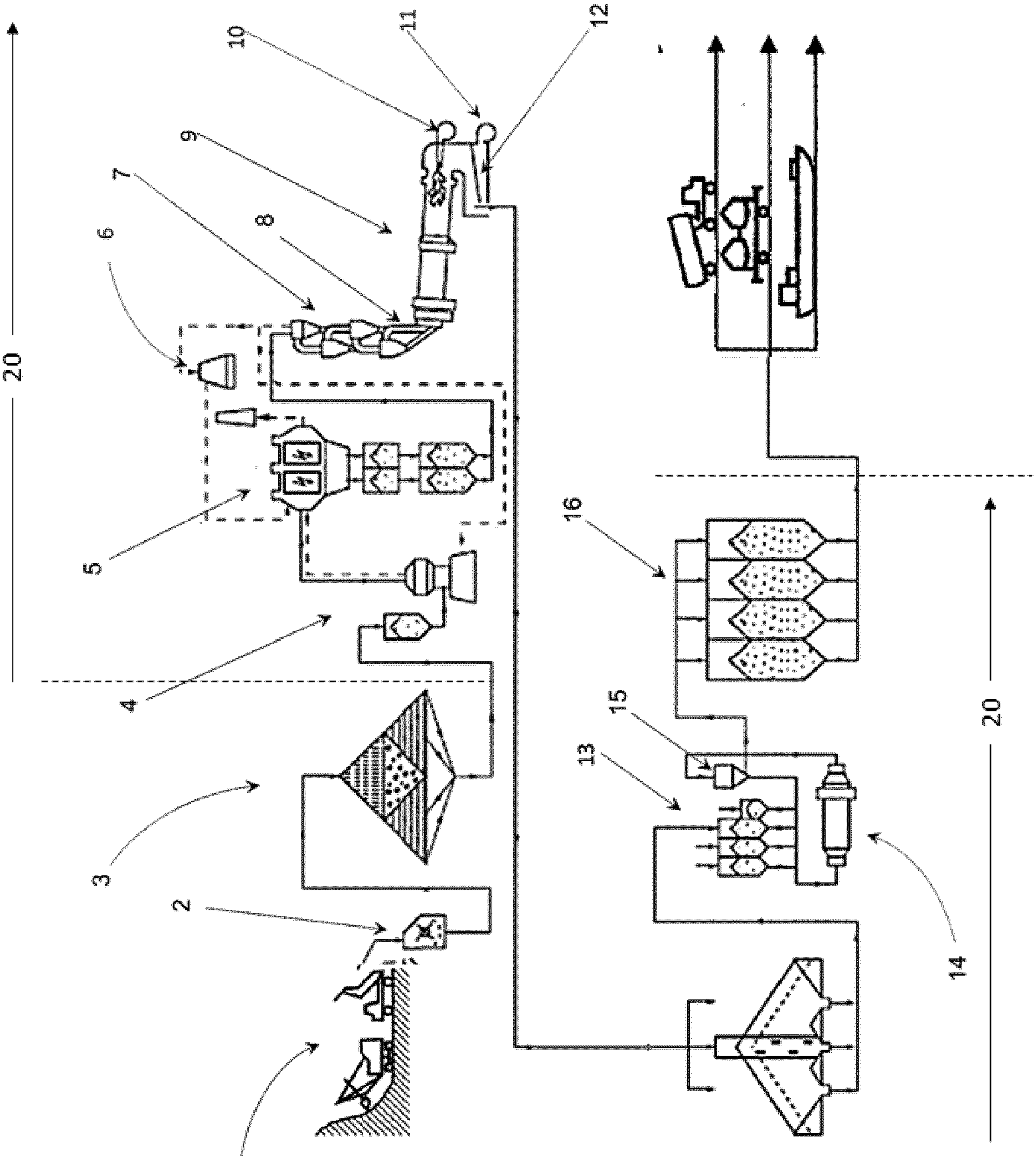


Fig.1

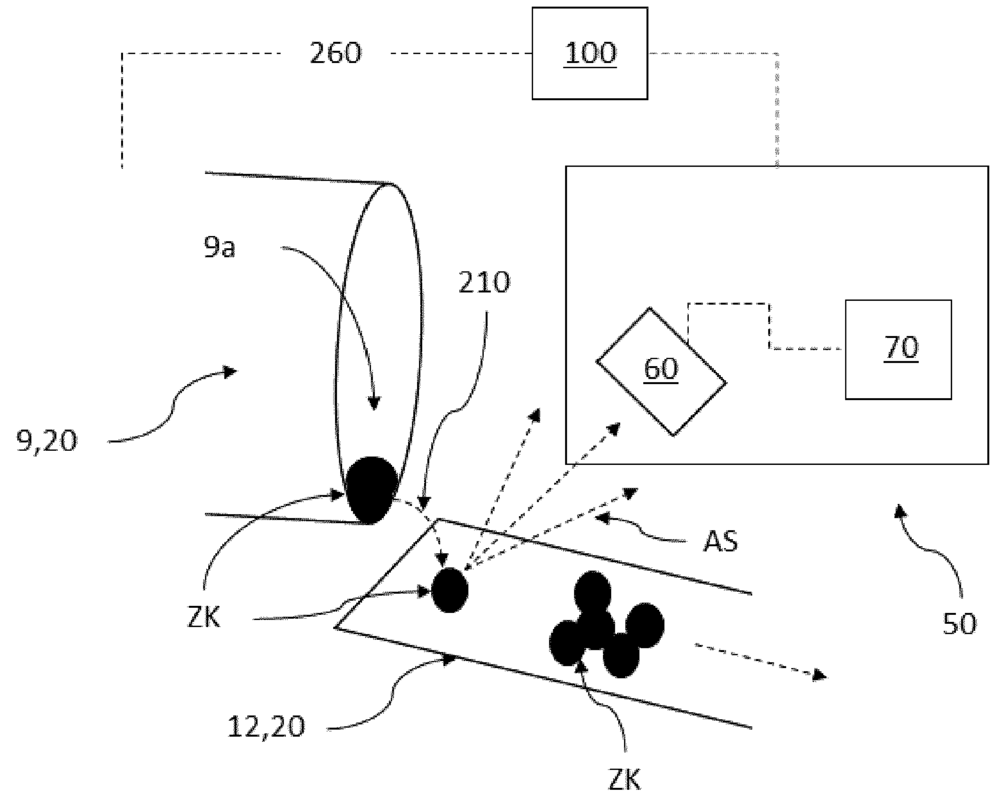
260
100
9a
210
60
70
9,20
AS
ZK
50
12,20
ZK

Fig.2

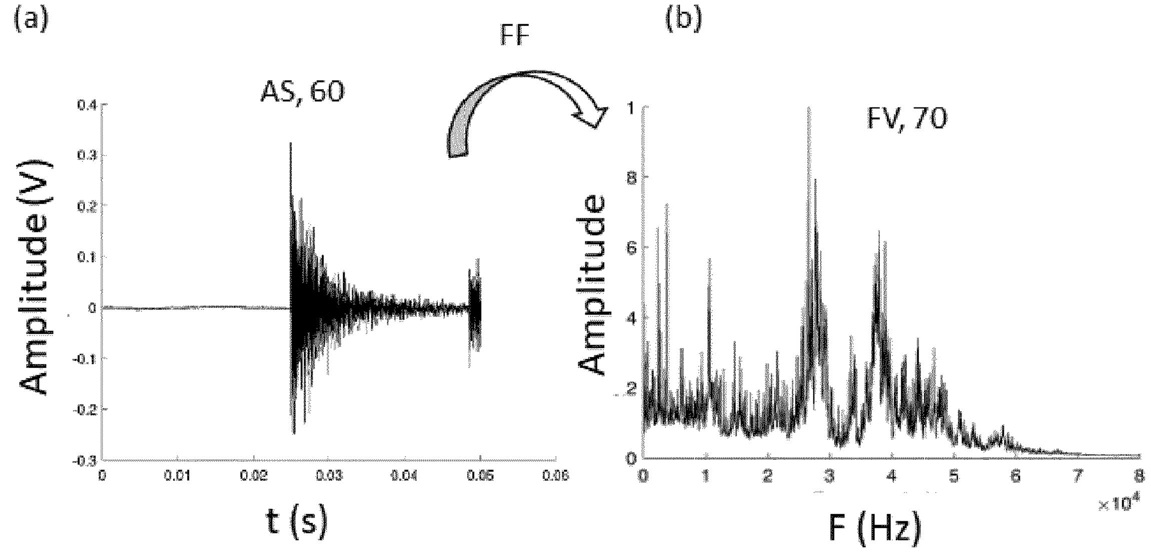
(a)
FF
(b)
AS, 60
FV, 70
Amplitude (V)
t (s)
Amplitude
F (Hz)

Fig.3

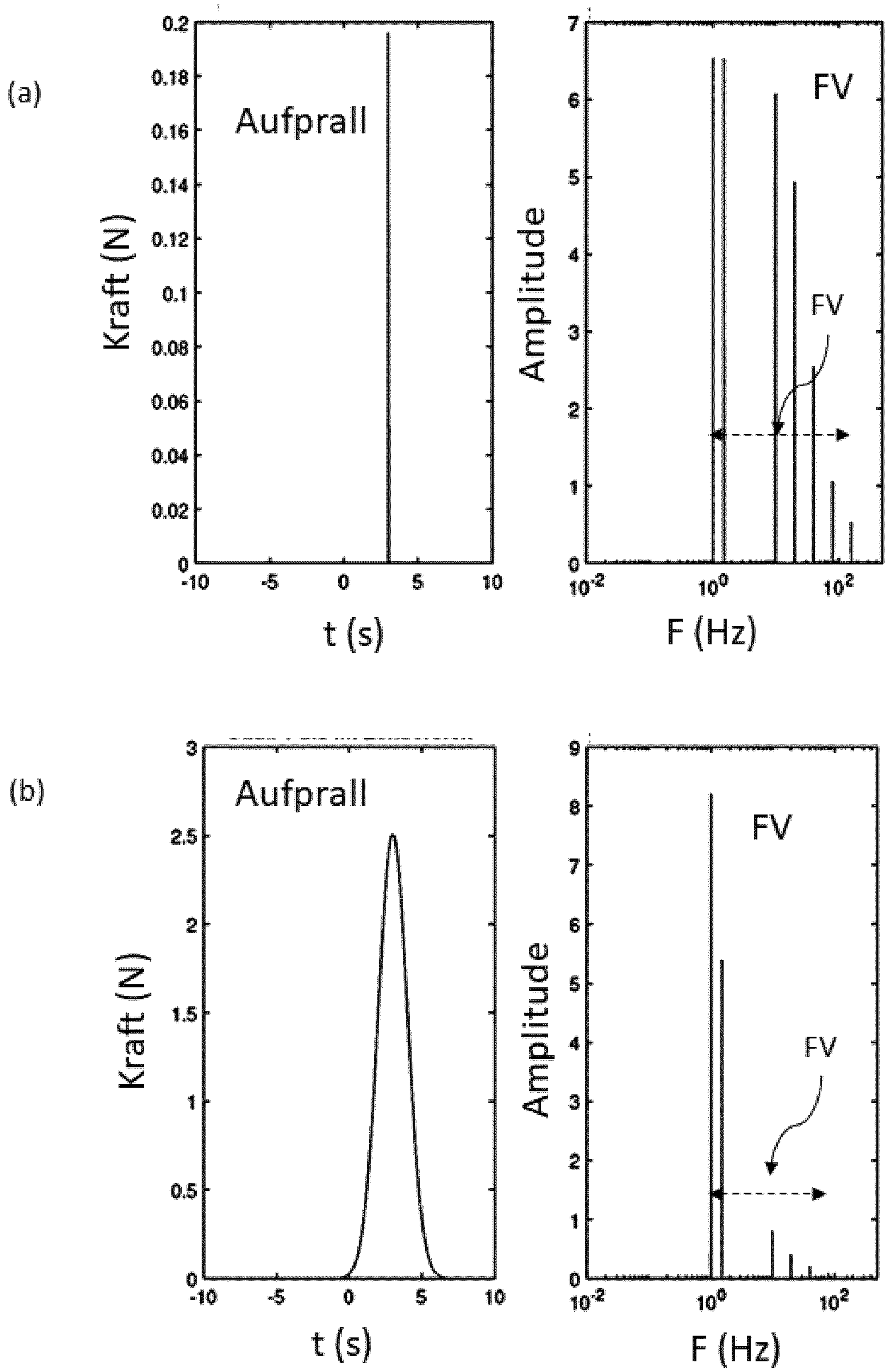
(a)
Aufprall
Kraft (N)
t (s)
FV
FV
Amplitude
F (Hz)
(b)
Aufprall
Kraft (N)
t (s)
FV
FV
Amplitude
F (Hz)

Fig.4

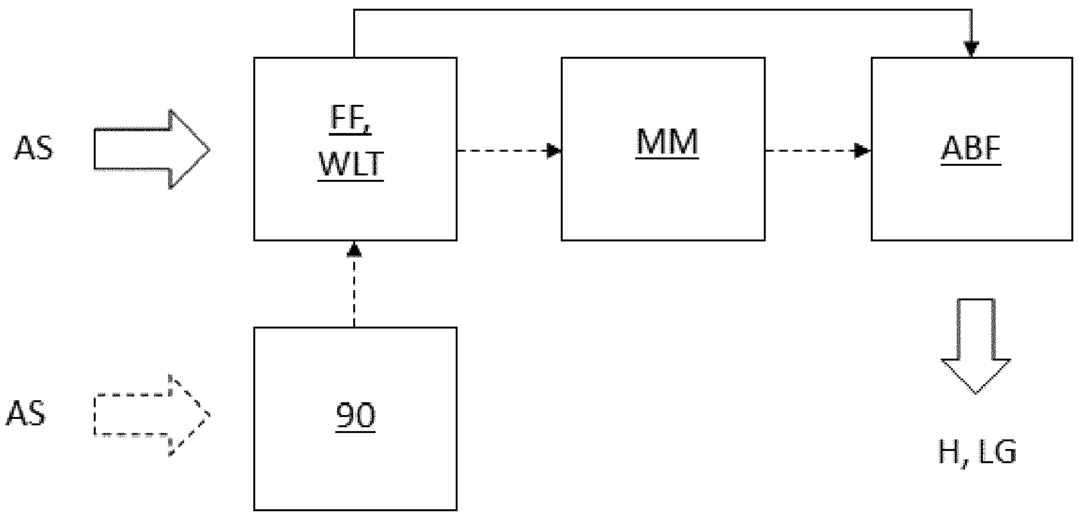


Fig.5

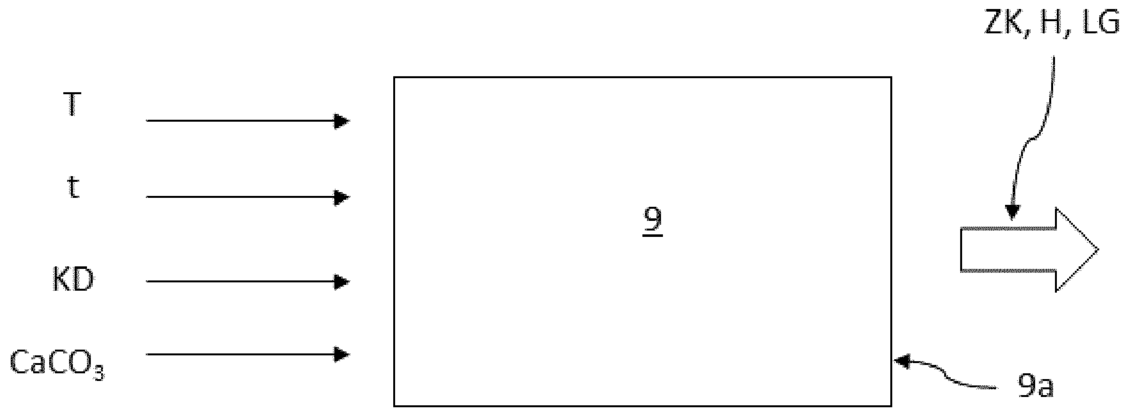


Fig.6

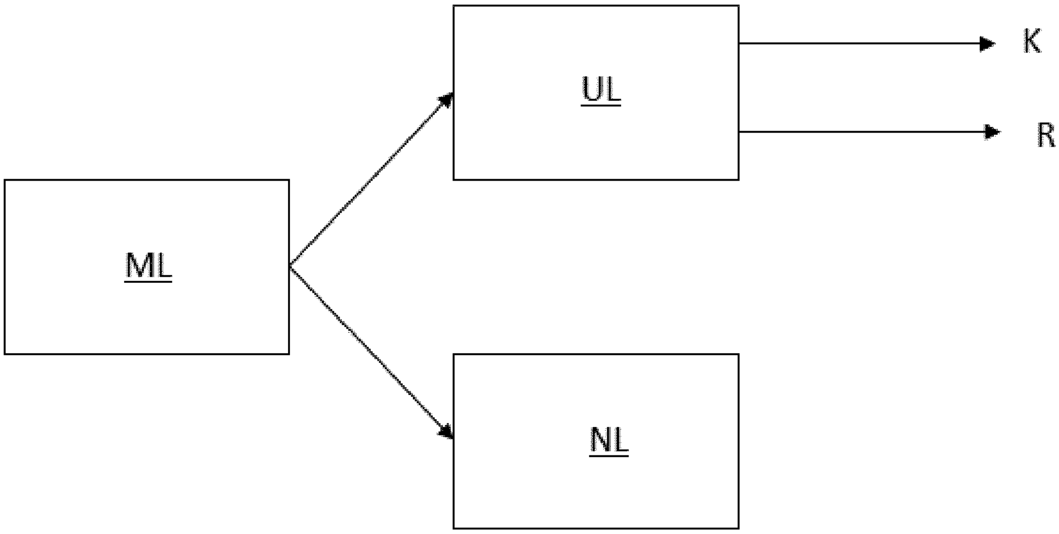


Fig.7

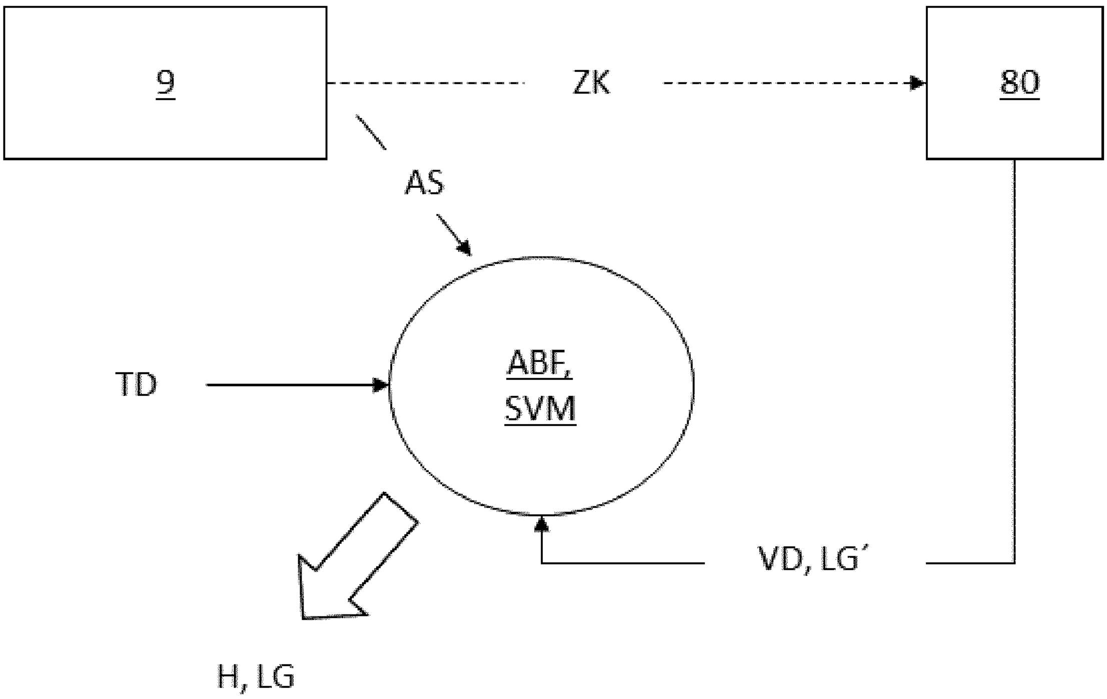


Fig.8

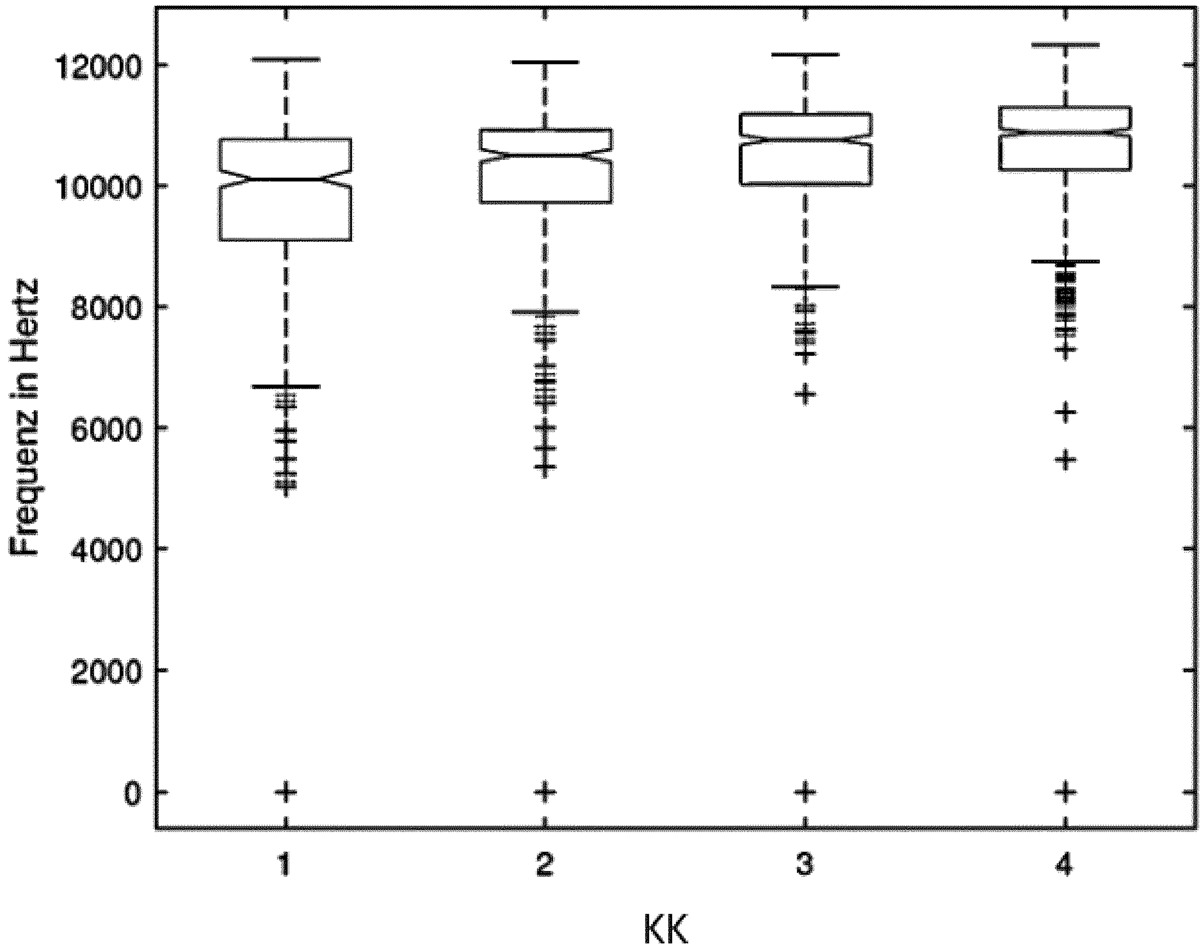
12000
10000
8000
6000
4000
2000
0
Frequenz in Hertz
1
2
3
4
KK

Fig.9

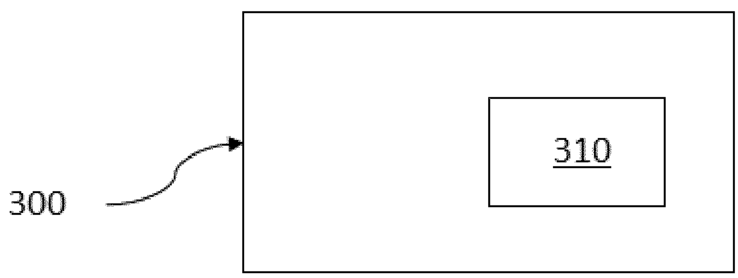
300
310

Fig.12

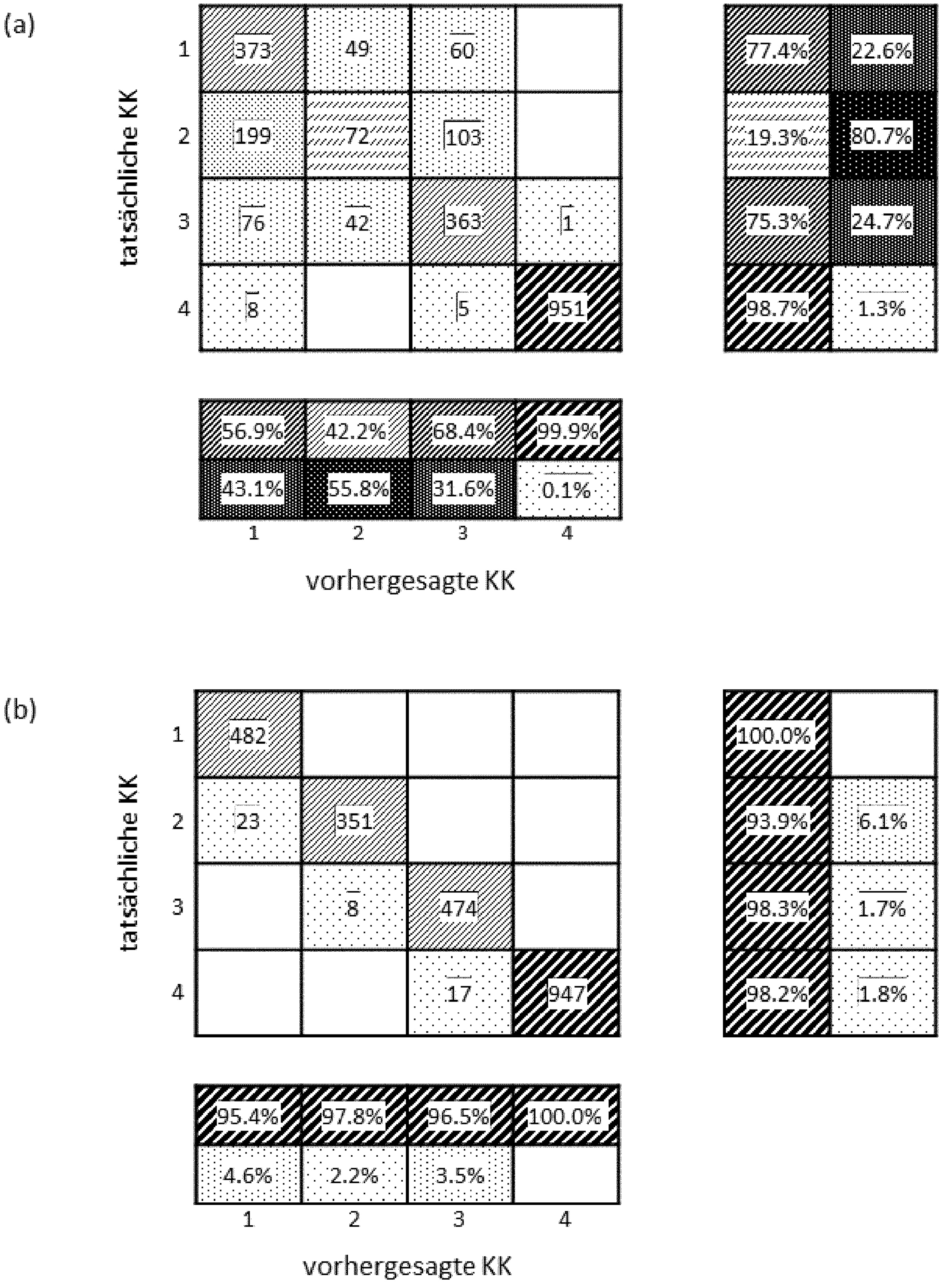
(a)
tatsächliche KK
1
2
3
4
373
49
60
199
72
103
76
42
363
1
8
5
951
77.4%
22.6%
19.3%
80.7%
75.3%
24.7%
98.7%
1.3%
56.9%
42.2%
68.4%
99.9%
43.1%
55.8%
31.6%
0.1%
1
2
3
4
vorhergesagte KK
(b)
tatsächliche KK
1
2
3
4
482
23
351
8
474
17
947
100.0%
93.9%
6.1%
98.3%
1.7%
98.2%
1.8%
95.4%
97.8%
96.5%
100.0%
4.6%
2.2%
3.5%
1
2
3
4
vorhergesagte KK

Fig. 10

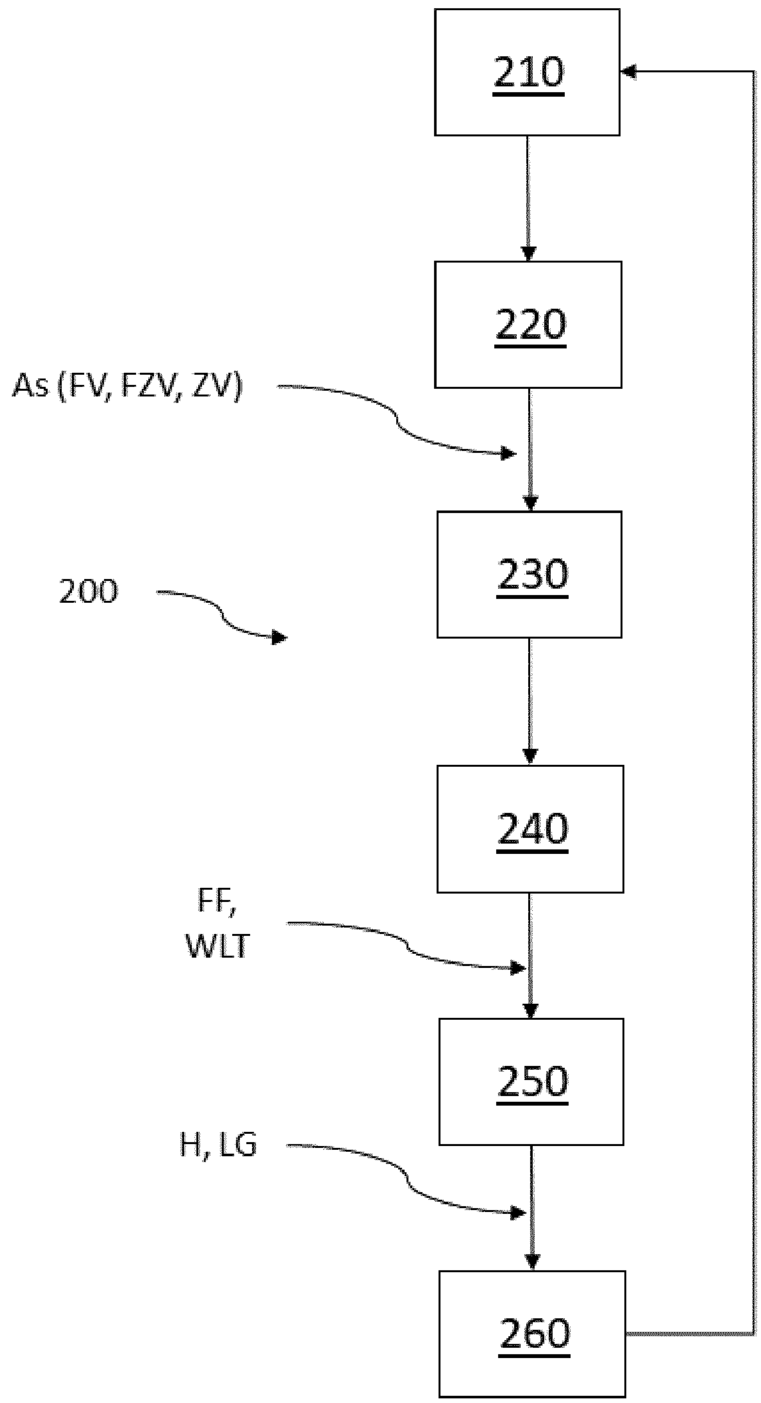


Fig.11

Europäisches Patentamt
European Patent Office
Office européen des brevets

# EUROPÄISCHER RECHERCHENBERICHT

Nummer der Anmeldung
EP 23 18 5000

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>Y | DE 25 43 470 A1 (POLYSIUS AG)<br>31. März 1977 (1977-03-31)<br>* das ganze Dokument *<br>----- | 1-3,13,<br>14,16<br>1-16 | INV.<br>G01N29/04<br>G01N29/14<br>G01N29/22<br>G01N29/44<br>G01N29/46<br>G01N33/38 |
| Y | WO 2012/086754 A1 (TAIHEIYO CEMENT CORP [JP]; KUROKAWA DAISUKE [JP] ET AL.)<br>28. Juni 2012 (2012-06-28)<br>* das ganze Dokument *<br>----- | 1-16 | |
| Y | PEDRO MONTES DE OCA: "Quality of clinker related to preheater performance",<br>2012 IEEE-IAS/PCA 53RD CEMENT INDUSTRY TECHNICAL CONFERENCE; SAN ANTONIO, TX ; 14-17 MAY 2012, IEEE, PISCATAWAY, NJ, USA,<br>14. Mai 2012 (2012-05-14), Seiten 1-7,<br>XP032186214,<br>DOI: 10.1109/CITCON.2012.6215698<br>ISBN: 978-1-4673-0284-5<br>* das ganze Dokument *<br>----- | 4 | |
| | | | RECHERCHIERTE SACHGEBIETE (IPC)<br>G01N |

Der vorliegende Recherchenbericht wurde für alle Patentansprüche erstellt

1

| Recherchenort | Abschlußdatum der Recherche | Prüfer |
|---|---|---|
| München | 4. Dezember 2023 | Roetsch, Patrice |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................................................
& : Mitglied der gleichen Patentfamilie, übereinstimmendes Dokument

EPO FORM 1503 03.82 (P04C03)

# ANHANG ZUM EUROPÄISCHEN RECHERCHENBERICHT ÜBER DIE EUROPÄISCHE PATENTANMELDUNG NR.

EP 23 18 5000

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-12-2023

| Im Recherchenbericht angeführtes Patentdokument | Datum der Veröffentlichung | Mitglied(er) der Patentfamilie | Datum der Veröffentlichung |
|---|---|---|---|
| DE 2543470 A1 | 31-03-1977 | BE 845242 A | 16-12-1976 |
| | | DE 2543470 A1 | 31-03-1977 |
| | | ES 451770 A1 | 16-08-1977 |
| | | FR 2325931 A1 | 22-04-1977 |
| | | ZA 764899 B | 31-08-1977 |
| WO 2012086754 A1 | 28-06-2012 | JP WO2012086754 A1 | 05-06-2014 |
| | | WO 2012086754 A1 | 28-06-2012 |

EPO FORM P0461

Für nähere Einzelheiten zu diesem Anhang : siehe Amtsblatt des Europäischen Patentamts, Nr.12/82